# EUROPEAN PATENT APPLICATION

(11) **EP 1 994 824 A1**
(43) Date of publication of application: **26.11.2008**
(21) Application number: 08162507.1
(22) Date of filing: 29.10.2002
(51) Int. Cl.: A01K 67/027, C12N 15/09, C12N 5/10

(54) **Chimeric non-human animal**

(30) Priority: 15.11.2001 JP 2001350481; 19.02.2002 JP 2002042321
(62) Divisional of application: 02777997.4
(71) Applicant: Kirin Pharma Kabushiki Kaisha, Tokyo 150-8011 (JP)
(72) Inventor: Tomizuka, Kazuma c/o Pharmaceutical Research Laboratories, Gunma 370-1295 (JP); Kakitani, Makoto c/o Pharmaceutical Research Laboratories, Gunma 370-1295 (JP); Yoneya, Takashi c/o Pharmaceutical Research Laboratories, Gunma 370-1295 (JP); Ishida, Isao c/o Pharmaceutical Research Laboratories, Gunma 370-1295 (JP)
(74) Representative: HOFFMANN EITLE

(57) **Abstract**

The present invention relates to a method for producing a chimeric non-human animal expressing a desired protein, and a chimeric non-human animal or an offspring thereof expressing a desired protein.

The present invention also relates to a method for analyzing the functions of a desired protein or a gene encoding the protein by comparing the phenotype of the above chimeric non-human animal with that of a corresponding wild-type animal.

SEQUENCE LISTING

<110> KIRIN BEER KABUSHIKI KAISHA

<120> Chimeric Non-Human Animal

<130> PH-1673PCT

<140> PCT/JP02/11236
<141> 2002-10-29

<150> JP 2001-350481
<151> 2001-11-15

<150> JP 2002-42321
<151> 2002-02-19

<160> 42

<170> PatentIn Ver. 2.0

<210> 1
<211> 38
<212> DNA
<213> Artificial Sequence

<220>
<223> Description of Artificial Sequence: synthetic
      oligonucleotide

<400> 1

atctcgagga accactttcc tgaggacaca gtgatagg                    38

<210> 2

<211> 38

<212> DNA

<213> Artificial Sequence

<220>

<223> Description of Artificial Sequence: synthetic

      oligonucleotide

<400> 2

atgaattcct aacactcatt cctgttgaag ctcttgac                    38

<210> 3

<211> 32

<212> DNA

<213> Artificial Sequence

<220>

<223> Description of Artificial Sequence: synthetic

      oligonucleotide

<400> 3

atgaattcag acaaaggtcc tgagacgcca cc                          32

<210> 4

<211> 42

<212> DNA

<213> Artificial Sequence

<220>

<223> Description of Artificial Sequence: synthetic
      oligonucleotide

<400> 4

atggatcctc gagtcgactg gatttcaggg caactaaaca tt                    42

<210> 5

<211> 32

<212> DNA

<213> Artificial Sequence

<220>

<223> Description of Artificial Sequence: synthetic
      oligonucleotide

<400> 5

atgaattcgc ccctctccct cccccccccc ta                                32

<210> 6

<211> 38

<212> DNA

<213> Artificial Sequence

<220>

<223> Description of Artificial Sequence: synthetic
      oligonucleotide

<400> 6
atgaattcgt cgacttgtgg caagcttatc atcgtgtt                    38

<210> 7
<211> 8
<212> DNA
<213> Artificial Sequence

<220>
<223> Description of Artificial Sequence: SalI-recognition sequence

<400> 7
agtcgaca                                                     8

<210> 8
<211> 14
<212> DNA
<213> Artificial Sequence

<220>
<223> Description of Artificial Sequence: SalI-recognition sequence

<400> 8
aatttgtcga ctgc                                              14

<210> 9

<211> 37

<212> DNA

<213> Artificial Sequence

<220>

<223> Description of Artificial Sequence: synthetic
      oligonucleotide

<400> 9

ccgctcgagc ggccaccatg gagctgactg atttgct                                37

<210> 10

<211> 35

<212> DNA

<213> Artificial Sequence

<220>

<223> Description of Artificial Sequence: synthetic
      oligonucleotide

<400> 10

ccgctcgagc ggctatgttt cctgagacaa attcc                                  35

<210> 11

<211> 45

<212> DNA

<213> Artificial Sequence

<220>

<223> Description of Artificial Sequence: synthetic
      oligonucleotide

<400> 11
ccctcgagcc accatgacaa cacccagaaa ttcag                                45

<210> 12
<211> 29
<212> DNA
<213> Artificial Sequence

<220>
<223> Description of Artificial Sequence: synthetic
      oligonucleotide

<400> 12
gggtcgactt aaggagagct gtcattttc                                       29

<210> 13
<211> 34
<212> DNA
<213> Artificial Sequence

<220>
<223> Description of Artificial Sequence: synthetic
      oligonucleotide

<400> 13
atctcgagcc accatgttgg gggcccgcct cagg                                 34

<210> 14

<211> 47

<212> DNA

<213> Artificial Sequence

<220>

<223> Description of Artificial Sequence: synthetic
      oligonucleotide

<400> 14

atctcgagct aatgatgatg atgatgatgg atgaacttgg cgaaggg                47

<210> 15

<211> 42

<212> DNA

<213> Artificial Sequence

<220>

<223> Description of Artificial Sequence: synthetic
      oligonucleotide

<400> 15

ggaattcaga catgataaga tacattgatg agtttggaca aa                     42

<210> 16

<211> 44

<212> DNA

<213> Artificial Sequence

<220>
<223> Description of Artificial Sequence: synthetic
      oligonucleotide

<400> 16
cccaagcttt aatcagccat accacatttg tagaggtttt actt                    44

<210> 17
<211> 44
<212> DNA
<213> Artificial Sequence

<220>
<223> Description of Artificial Sequence: synthetic
      oligonucleotide

<400> 17
cccaagcttg aattaaacag tttcagggca catgaaatac tgag                    44

<210> 18
<211> 43
<212> DNA
<213> Artificial Sequence

<220>
<223> Description of Artificial Sequence: synthetic
      oligonucleotide

<400> 18

gctctagatt tgtctttgaa ttttggtccc tagctaatta ctg                    43

<210> 19

<211> 44

<212> DNA

<213> Artificial Sequence

<220>

<223> Description of Artificial Sequence: synthetic

      oligonucleotide

<400> 19

cccaagcttt ggtgattatt cagagtagtt ttagatgagt gcat                   44

<210> 20

<211> 43

<212> DNA

<213> Artificial Sequence

<220>

<223> Description of Artificial Sequence: synthetic

      oligonucleotide

<400> 20

gctctagatt tgtctttgaa ctttggtccc tagctaatta cta                    43

<210> 21

<211> 45

<212> DNA

<213> Artificial Sequence

<220>

<223> Description of Artificial Sequence: multiple cloning site

<400> 21

gctctagagt cgacgcggcc gcggccggcc ggcgcgccac tagtc 45

<210> 22

<211> 45

<212> DNA

<213> Artificial Sequence

<220>

<223> Description of Artificial Sequence: multiple cloning site

<400> 22

gactagtggc gcgccggccg gccgcggccg cgtcgactct agagc 45

<210> 23

<211> 42

<212> DNA

<213> Artificial Sequence

<220>

<223> Description of Artificial Sequence: synthetic
oligonucleotide

<400> 23

gactagtaga caaaggtcct gagacgccac caccagctcc cc                    42

<210> 24

<211> 43

<212> DNA

<213> Artificial Sequence

<220>

<223> Description of Artificial Sequence: synthetic

      oligonucleotide

<400> 24

gaagatctca agtgcaaaga ctcactttat tgaatatttt ctg                   43

<210> 25

<211> 42

<212> DNA

<213> Artificial Sequence

<220>

<223> Description of Artificial Sequence: synthetic

      oligonucleotide

<400> 25

ggaattcaga caaaggtcct gagacgccac caccagctcc cc                    42

<210> 26

<211> 44

<212> DNA
<213> Artificial Sequence

<220>
<223> Description of Artificial Sequence: synthetic
      oligonucleotide

<400> 26
cccaagcttg cctcctcaaa cctaccatgg cccagagaaa taag                44

<210> 27
<211> 20
<212> DNA
<213> Mus musculus

<400> 27
gactcctggt gggcgtgctc                                           20

<210> 28
<211> 20
<212> DNA
<213> Mus musculus

<400> 28
ggtgccatct acatgaccat                                           20

<210> 29
<211> 22
<212> DNA

<213> Artificial Sequence

<220>
<223> Description of Artificial Sequence: synthetic
      oligonucleotide

<400> 29
aattaaccct cactaaaggg aa                                          22

<210> 30
<211> 22
<212> DNA
<213> Mus musculus

<400> 30
caagcaatgg ggaagtgtct gg                                          22

<210> 31
<211> 22
<212> DNA
<213> Mus musculus

<400> 31
cggctacagc caggaccagc ta                                          22

<210> 32
<211> 24
<212> DNA
<213> Artificial Sequence

<220>

<223> Description of Artificial Sequence: synthetic
      oligonucleotide

<400> 32
gtaatacgac tcactatagg gcga                                          24

<210> 33
<211> 59
<212> DNA
<213> Artificial Sequence

<220>
<223> Description of Artificial Sequence: synthetic
      oligonucleotide

<400> 33
ataagaatgc ggccgctaaa ctatagcatc cactgggaat caacatctga gacatccta  59

<210> 34
<211> 47
<212> DNA
<213> Artificial Sequence

<220>
<223> Description of Artificial Sequence: synthetic
      oligonucleotide

<400> 34

cgggccggcc cgcgggactt ttaaagggtg gtggtgtgac atcaagc 47

<210> 35

<211> 43

<212> DNA

<213> Artificial Sequence

<220>

<223> Description of Artificial Sequence: synthetic

oligonucleotide

<400> 35

ggcgcgccca ctgctagagc ctatccagac acttccccat tgc 43

<210> 36

<211> 47

<212> DNA

<213> Artificial Sequence

<220>

<223> Description of Artificial Sequence: synthetic

oligonucleotide

<400> 36

ccgctcgagc ggtgttccag actgaccacc tttcaacaaa gagattc 47

<210> 37

<211> 28

<212> DNA
<213> Artificial Sequence

<220>
<223> Description of Artificial Sequence: synthetic
      oligonucleotide

<400> 37
tcgagtcgcg acaccggcgg gcgcgccc                                    28

<210> 38
<211> 28
<212> DNA
<213> Artificial Sequence

<220>
<223> Description of Artificial Sequence: synthetic
      oligonucleotide

<400> 38
tcgagggcgc gcccgccggt gtcgcgac                                    28

<210> 39
<211> 29
<212> DNA
<213> Artificial Sequence

<220>
<223> Description of Artificial Sequence: synthetic

oligonucleotide

<400> 39
ggccgcttaa ttaaggccgg ccgtcgacg                                    29

<210> 40
<211> 29
<212> DNA
<213> Artificial Sequence

<220>
<223> Description of Artificial Sequence: synthetic
      oligonucleotide

<400> 40
aattcgtcga cggccggcct taattaagc                                    29

<210> 41
<211> 30
<212> DNA
<213> Mus musculus

<400> 41
tcagtctaaa tggcaggctt acagacatcc                                   30

<210> 42
<211> 30
<212> DNA
<213> Mus musculus

<400> 42

tgaggcagat cattccatct tgtcaagacc 30

## Description

### TECHNICAL FIELD

The present invention relates to a method for producing a chimeric non-human animal expressing a desired protein, and a chimeric non-human animal or an offspring thereof expressing a desired protein.

The present invention further relates to a method for analyzing the functions of a desired protein or a gene encoding such protein by comparing the phenotype of the above chimeric non-human animal with that of a corresponding wild-type animal.

### BACKGROUND ART

The determination of the entire nucleotide sequence of the human genome (International Human Genome Sequencing Consortium, Nature, 409: 860-921, 2001), the historic achievement of research, has provided at the same time a new research theme for elucidating the functions of a large number of novel genes. Taking as an example human chromosome 22 (Dunham et al., Nature, 402: 489-495, 1999), which is the second-smallest chromosome among the 24 human chromosomes, and whose entire nucleotide sequence was the first to be determined, the presence of a total of 545 genes (pseudogenes excluded) has been inferred. The breakdown thereof is: 247 genes having known nucleotide sequences and amino acid sequences; 150 novel genes showing homology with the known genes; and 148 novel genes homologous to sequences with unknown functions registered with the Expressed Sequence Tag (EST) database. In addition, according to analyses made by software (GENESCAN) predicting genes directly from genomic sequences, 325 further novel genes whose transcription products have remained unconfirmed have been predicted (Dunham *et al., supra*). Elucidation of *in vivo* functions of genes and the products thereof (proteins) is not only important for understanding the programs of life activities, but also can lead to development of new pharmaceuticals to overcome various human diseases. In other words, technological development for efficient functional analyses of novel genes is a big issue in the fields of life science and medical research in the post genome era.

As the most direct techniques to examine the in vivo functions of mammalian genes, transgenic (Tg) mice wherein a foreign gene is inserted into a mouse chromosome and the overexpression thereof is caused (Gordon et al., Proc. Natl. Acad. Sci. U.S.A., 77: 7380-7384, 1980), knock-out (KO) mice wherein an endogenous gene is disrupted (Shinichi Aizawa, Bio Manual Series 8, Gene Targeting, YODOSHA, 1995), and the like have been widely utilized.

Tg mice are generally produced by directly injecting purified DNA containing an expression unit comprising the cDNA of a gene to be expressed (hereinafter referred to as a transgene), an appropriate promoter, and a poly A addition site into a mouse fertilized egg nucleus (Gordon *et al., supra).* Since it has been shown that a Tg mouse having a rat-derived growth hormone gene introduced therein grows to a size nearly double that of a normal mouse (Palmiter et al., Nature, 300: 611-615, 1982), Tg mice have been utilized to examine the *in vivo* functions of numerous genes (and their products, proteins). A transgene in a Tg mouse may be expressed to a greater degree at a site differing from a site at which the gene is normally expressed by the action of a promoter or the like differing from the normal expression regulatory system. This may be generally referred to as ectopic overexpression. A response at the whole body level that is induced by the ectopic overexpression of a transgene can be said as highly important information for inferring the *in* vivo functions of the protein.

The establishment of mouse embryonic stem cells (ES cells) having pluripotency, the development of techniques to alter a target gene by homologous recombination, and the like have been achieved since the middle of the 1980's. Around 1990, the production of a mouse wherein a specific gene was artificially disrupted (KO mouse) became possible (Capecchi, Trends Genet., 5: 70-76, 1989). For example, the fact that the homozygote of a KO mouse, wherein transcription factor GATA-2 that is expressed in blood stem cells and vascular endothelia has been disrupted, dies in the early developmental stage because of anemia has indicated the importance of this transcription factor in hematopoiesis (Tsai et al., Nature, 371: 221-226, 1994). Many KO mice have been produced by many researchers to date. The analytical results have provided not only important information in a wide variety of fields ranging from basic biology to clinical medicine, but also many human disease model animals. Today, the use of KO mice is still the most widely used technique for elucidating the *in vivo* functions of a gene.

However, both the technique using Tg mice and the technique using KO mice require considerable time and effort, even for handling a single gene. This problem has not been addressed to date. Thus, it has been considered that the use of these techniques is unreaslistic for the exhaustive examination of the functions of many novel genes found from the above-mentioned genomic sequence information.

In a further simple method for elucidating the functions of a gene, a virus vector or a plasmid DNA containing the expression unit of a target gene has been administered to an appropriate site in an animal, so as to examine a phenotype induced by the following gene expression (Cannizzo el al., Nature Biotechnol., 15: 570-573, 1997; Ochiya et al., Nature Medicine, 5: 707-710, 1999). However under the current circumstances, such a method is not satisfactory as a technique for analyzing novel genes with unknown functions, because it has many problems in terms of introduction efficiency, antigenicity, expression stability, and the like.

Furthermore, when the functions of a humoral factor or a membrane protein are analyzed, administration of a recombinant protein or a specific antibody to an experimental animal is an effective means for functional analysis. However, preparing purified proteins or obtaining antibodies for various types of gene products with unknown functions cannot be said to be a realistic means, as in the cases of producing the above Tg and KO mice.

To analyze the functions of secretory proteins such as hormones, growth factors, and cytokines, or proteins prepared by altering membrane proteins to be secretory, an effective means involves artificially increasing the effective concentration of such a protein in blood, or the local effective concentration of the same, and then examining the effect. As a method for increasing effective concentrations, the above-described ectopic expression in Tg mice, administration of a recombinant protein, gene transfer into an individual, or the like has been conventionally utilized. Above all, overexpression of a gene in Tg mice is the most widely employed method. This method has been utilized for examining the effect of the overexpression of a gene with known functions (Palmiter *et al., supra*), or for identifying the functions of a novel gene in few cases. Sirauonet et al. (Cell, 18: 309-319, 1997) have produced Tg mice expressing the cDNA encoding a novel gene with unknown functions under the regulation of ApoE gene promoter that is expressed specifically in the liver to analyze the phenotype, thereby discovering osteoprotegrin (OPG) exhibiting an effect of increasing bone quantity through the high level expression thereof.

However, a significant problem in the production of Tg mice is that a transgene is inserted randomly into a host chromosome, and the expression is affected by the insertion position. For example, even when the promoter of a housekeeping gene showing constitutive strong expression is utilized, an expected animal expressing the transgene at high levels cannot be easily obtained. In general, it is thought that the first generation individuals (F0 individuals), which are born from fertilized eggs containing foreign DNA injected therein, carry the introduced DNA at an efficiency approximately ranging from 10% to 20%. Furthermore, it is thought that individuals showing expression as expected account for approximately 0% to 20% of the DNA-introduced individuals. Moreover, a problem that the number of copies of a transgene is unable to control may cause an event wherein the transgenes are inactivated because of the insertion of multiple copies thereof (Garrick et al., Nature Genet., 18: 56-59, 1998). Thus this is a reason causing a difficulty in obtaining individuals for expression. Furthermore, a requirement of maintaining and breeding many mice for a long period to establish a Tg mouse strain expressing a transgene is also a big problem. As described above, the probability of obtaining an expected individual for expression in the production of Tg mice is generally several percent or less, so that there is a need to produce many F0 mice. Furthermore, it is inappropriate as described below to directly use F0 mice, that have been confirmed to carry a transgene by the analysis or the like of DNA derived from the tail tissue, for expression analysis or phenotype analysis. Generally, detailed analysis can be conducted only when F1 mice are obtained by crossing with wild-type mice.

It is considered possible to solve the above problems, the random insertion into the host chromosome of the Tg mouse and the uncontrollable number of copies, by the use of gene targeting in mouse ES cells (Shinichi Aizawa, *supra;* Capecchi, *supra*). Specifically, the effect of insertion position on expression can be avoided by inserting (knock-in) a target gene downstream of an endogenous gene promoter, and then causing the expression of the gene under the regulation of the promoter. Knock-in has been conventionally utilized mainly for cases such as those shown below.
(1) A foreign gene (e.g., marker gene) is inserted such that it is substituted with a target gene. In this case, the target gene is disrupted. Instead, the marker gene or the like is expressed with specificity similar to that of the target gene. This is used for a case, for example, when a phenotype resulting from gene disruption is examined, and the specificity of a promoter is examined at the same time.
(2) The normal type of a target gene is substituted with a variant type (or an analogous gene) thereof. This technique is used for producing a disease model mouse by the expression of a variant of a specific gene, or for examining the interchangeability of gene functions.

As an early report concerning (1) above, Le Moucllic et al. (Proc. Natl. Acad. Sci. USA, 87: 4712-4716, 1990) have produced chimeric mice from ES cells having the *Escherichia coli* LacZ gene inserted into the Hox-3.1 gene locus, and showed that the LacZ gene product is expressed with specificity similar to that of Hox-3.1. Moreover, Jin et al. (BBRC. 270: 978-982, 2000) have inserted not only the LacZ gene in a manner similar to the above report, but also the Cre gene that has been ligated to an internal ribosomal entry site (IRES; Jang et al., J. Virol., 62: 2636-2643, 1988) downstream of the Lac Z gene into the Emx1 gene that is expressed specifically in the cerebral cortex, thereby showing that the Cre protein is expressed in the cerebral cortex.

In the meantime, there is a report as an example of (2) above that by the insertion of a recombinant functional heavy chain VDJ variable region gene fragment into a specific region of an antibody heavy chain gene, a mouse producing antibodies of all the classes having a common variable region has been produced. In a mouse wherein a VDJ fragment having autoantigenic reactivity by itself, or having autoantigenic reactivity by a combination with a specific light chain has been inserted upstream of a heavy chain constant region, B cells have developed and differentiated normally, and antibody production closely resembling physiological antibody production except for a single heavy chain variable region being expressed has been observed (Sonoda et al., Immunity, 6: 225-233, 1997).

As an example differing from both (1) and (2) above, there is a recent report that a G418-resistance gene ligated to IRES has been inserted into the downstream untranslated region (between the termination codon and the polyA addition site of the α1(I) procollagen (COL1A1) gene) of the α1(I) procollagen (COL1A1) gene of a sheep-foetus-derived fibroblast (McCreath et al., Nature, 405: 1066-1069, 2000). The sheep has been produced by transferring a cell nucleus derived from the above fibroblast clone into the unfertilized egg of a sheep. In this case, the COL1A1 gene is normally expressed, and G418-resistance gene is further expressed with similar specificity.

As described above, knock-in is considered to be an effective technique to overcome the disadvantages of the method for producing Tg mice. However, there may still be some problems when the method is utilized for exhaustive gene analysis. First, the frequency of homologous recombination in mouse ES cells is normally several percent or less. Considerable effort is required for obtaining only a homologous recombinant clone wherein knock-in has been conducted downstream of an appropriate promoter for one type of a gene. Furthermore, in chimeric mice produced from a knock-in ES cell line as described below, the chimerism (rate of the contribution of knock-in ES cells) differs in every individual. In this case, the ratio of cell populations expressing a transgene to other cell populations varies depending on each individual and each tissue. This ratio may not be consistent with chimerism as determined from coat color, so that an understanding of the results of phenotype analysis is accompanied by difficulties. Accordingly, utilization of mouse offspring, to which above knock-in gene locus is transmitted, is desired.

For both Tg and knock-in described above, it is generally desired to analyze individuals after the transmission of a transgene to offsprings. However, in consideration of the experimental period, facilities, and effort required for keeping mice, it is desired to be able to conduct analysis using the first generation (in the case of Tg, F0 individuals, and in the case of knock-in, chimeric individuals) for efficient analyses of various types of genes. In the case of F0 individuals of Tg that rely on random insertion, the use of the second generation, the F1 individuals, is essential, because it is impossible to obtain again individuals wherein transgenes have been inserted into the same site in the same number of copies. On the other hand, in the case of knock-in, many chimeric individuals can be easily obtained from one type of ES clones. However, as described above, normally the chimerism differs largely depending on the individuals used, and individuals having a high chimerism that demonstrates the high level expression of a transgene cannot be easily obtained in large numbers. In addition, the chimerism is normally determined by coat color. However, the chimerism of tissues where a transgene is expressed may not always be consistent therewith.

In recent years, a system has been developed wherein all the cells of a tissue of a chimeric mouse are derived from ES cells. This is referred to as blastocyst complementation (hereinafter described as the BC method). There have been successful examples of the BC method in T or B cells of the immune system (Chen et al., Proc. Natl. Acad. Sci. U.S.A., 90: 4528-4532, 1993), lens (Liegeois et al., Proc. Natl. Acad. Sci. U.S.A., 93: 1303-1307,1996), and the like. For example, the BC method for T or B cells has been developed to examine the functions of a gene that will be lethal when knock-out is conducted by a general method in T or B cells. KO mice having a knocked-out RAG2 gene that is essential for site-specific recombination taking place in early development of T or B cells are unable to produce functional T or B cells. When a chimera is produced using a RAG2-KO mouse embryo as a host with a wild-type ES cell, T or B cells (derived from ES cells) nearly at the normal level are observed in many chimeric mice regardless of the chimerism. That is, by the use of an ES cell wherein embryonic lethal genes in both alleles have been knocked out (KO), only the functions of the gene in T or B cells can be examined in a chimeric mouse.

The entire nucleotide sequence of the human genome has been determined. Desired now is a system allowing exhaustive *in vivo* functional analyses to be conducted on a large number of novel genes. For this purpose, it is required to be able to reliably, easily, and simultaneously produce many types of animals expressing transgenes at high levels. As described above, since conventional methods using such as Tg mice or knock-in are unable to satisfy this requirement, it has been thought that there is no conclusive method for conducting functional analyses of a large number of genes obtained from genomic sequence information. Besides, it has also been considered difficult to directly utilize an individual for screening for functions of a large number of genes. Among novel genes provided by the human genome information, genes encoding secretory proteins having homology with cytokines, growth factors, hormones and the like can be said to be very interesting research objects in that they themselves can be pharmaceuticals. Hence, it is inferred that development of new efficient techniques for analyzing the *in vivo* functions of genes encoding secretory proteins and products thereof can lead to great advances in the development of pharmaceuticals for treating human diseases.

As described above, mutant mice (knock-out/KO mice) that are produced from embryonic stem (ES) cells wherein a specific gene has been disrupted by gene targeting are widely utilized as an essential tool in elucidation of the functions of genes (Shinichi Aizawa, *supra*).

Now, 10 or more years after KO mice were produced for the first time, the production of KO mice is recognized by many researchers as a procedure that requires much effort and time. One major reason for this is difficulty in obtaining homologous recombinants using ES cells. A knock-out (KO) vector has a structure wherein, in a target gene genomic DNA fragment of a certain size (5 kb) or greater, a drug-resistance marker is generally present after substitution with a target gene exon or insertion into the exon. By the electric pulse method employed in general protocols, colonies that are resistant to the above drug are obtained at rates of 1 out of 10,000 to 1,000,000 ES cells used. However, most resistant clones are prepared by inserting an introduction vector randomly into a mouse chromosome. Thus, it is thought that homologous recombination occurs in approximately 1 out of 100 to 10000 such clones. To improve the rate of homologous recombinants to clones with random insertion, various studies have been conducted. For example, it has been reported that a longer length of the genomic DNA of a homologous region contained in a vector is preferred (Deng & Capecchi, Mol. Cell. Biol., 12: 3365-71, 1992), or it has been reported that the use of the genomic DNA (isogenic DNA) from a mouse strain that is the same as that of a mouse ES cell to be subjected to targeting is preferred (Deng & Capecchi, *supra*). Furthermore, in the method that is currently most widely used, a KO vector containing a negative selection marker outside the genomic DNA of a homologous region is used in addition to the above selection marker. This method utilizes an event whereby a toxic negative marker is expressed in a clone prepared by random insertion so as to cause the cell to die; however, this does not occur in a homologous recombinant. As negative selection markers, the HSV-tk gene (a thymidine analog such as gancielovir, FIAU, or the like is required to be added to a medium) reported by Mansour et al. (Nature, 336: 348-352, 1988), DT-A (diphtheria toxin A chain) reported by Yagi et al. (Anal. Biochem., 214: 77-86, 1993), and the like are known. When this negative selection method functions as theorized, a result predicted is that all colonies would be homologous recombinants. However in actual cases, the results vary widely among reports, and the resulting enrichment effect is thought to be, in general, several times greater than those of other cases. Even under current actual situations where the negative selection method is generally employed in knock-out experiments, obtaining 100 to 1000 clones with random insertion and selecting homologous recombinants from these clones are carried out regularly.

In addition to the production of mice from variant ES cells, mammalian cell lines prepared by the alteration or disruption of genes by homologous recombination are themselves important materials for elucidating the functions of the genes. Furthermore, homologous recombination has been considered to be an ultimate therapy for diseases (hereditary diseases) caused by gene deletion or mutation. However, the ratio of homologous recombinants to clones with random insertion in mammalian cell lines or primary cultured cells is equivalent to, or lower than that in mouse ES. In application for the analysis of gene functions or gene therapy at the cellular level, improvement in the above ratio has been expected (Yanez & Poter, Gene Therapy, 5: 149-159, 1998). As attempts other than the above cases concerning improvement in the ratio of homologous recombinants to clones with random insertion, (1) high level expression, inactivation, inhibition, and the like of proteins (genes) involved in DNA recombination or repair, (2) specific damages to target genes, and the like have been examined. However, no method that can provide drastic improvement in the ratio of homologous recombinants in a chromosome gene, is simple, and has high reproducibility has been known (Yanez & Poter, *supra;* Vasquez et al. Proc. Natl. Acad. Sci. U.S.A., 98: 8403-8410, 2001).

### SUMMARY OF THE INVENTION

The purpose of the present invention is to provide a simple and highly reproducible method for analyzing gene functions.

In the present invention, by the utilization of gene targeting by homologous recombination, a desired gene is inserted into a gene locus for the expression in any cells and/or tissues, for example, a mouse Igκ gene locus, in a chimeric non-human animal. Gene targeting means altering a specific gene in a chromosome utilizing homologous recombination. Efficiently obtaining cells wherein homologous recombination has occurred is also important as an object to be achieved.

As a result of intensive studies to achieve the above objects, the present inventors have produced a chimeric mouse from a mouse ES cell wherein a structural gene encoding a secretory protein is inserted downstream of an immunoglobulin light chain gene, and a host embryo derived from a B-lymphocyte-deficient strain, and then found that in the B cell of the chimeric mouse, the above secretory protein is expressed at high levels. Furthermore, the present inventors have found a simple method for improving the ratio of homologous recombinants to clones with random insertion of a targeting vector in a mammalian cell, and thus have completed the present invention.

In a chimeric animal produced by the method of the present invention, an overexpression effect of the product derived from the introduced structural gene is observed regardless of the chimerism based on coat color. By the use of this system, a chimeric animal expressing a transgene more efficiently and more reliably at high levels than is possible with conventional methods can be obtained. This high efficiency when compared with that of conventional methods is based mainly on the fact that by the use of the B-lymphocyte-deficient host embryo, all the B lymphocytes of the chimeric animal are derived from the ES cells, regardless of the chimerism. In particular, the high efficiency obtained when the regulatory region of an immunoglobulin light chain gene is utilized is based on the following points:
(1) Homologous recombination occurs in Igκ gene locus at an efficiency of 5% or more.
(2) The expression system of immunoglobulin is utilized.
(3) The expression of immunoglobulin is very low in the early developmental stage, and shows an explosive increase at and after the weanling stage. Thus, even when a gene whose high level expression causes embryonic lethality is introduced, the functions of the gene in an adult can be examined.

The present invention is summarized as follows.

In a first aspect, the present invention provides a method for producing a chimeric non-human animal, which comprises the steps of:
1) preparing a pluripotent cell derived from a non-human animal containing a genome wherein a nucleic acid sequence encoding a desired protein is located so that the expression of the desired protein is regulated by the regulatory region of a gene that is expressed in at least a specific cell and/or tissue;
2) obtaining a chimeric embryo by injecting the pluripotent cell prepared in the above step 1 into a host embryo of a non-human animal strain that is deficient in the above specific cell and/or tissue;
3) transplanting the chimeric embryo obtained in the above step 2 to a foster mother of non-human animal of the same species; and
4) selecting a chimeric non-human animal expressing the desired protein in at least the above specific cell and/or tissue from offsprings obtained after the above transplantation step 3.

In one embodiment of the present invention, the above nucleic acid sequence encoding a desired protein is located downstream of the regulatory region of a gene that is expressed in a specific cell and/or tissue. In another embodiment, the above nucleic acid sequence encoding a desired protein is located downstream of an internal ribosomal entry site located downstream of the termination codon of a gene that is expressed in a specific cell and/or tissue.

In still another embodiment, a sequence containing an internal ribosomal entry site and the above nucleic acid sequence encoding a desired protein is located between the termination codon and a polyA signal region of a gene that is expressed in a specific cell and/or tissue.

In still another embodiment, a sequence containing a polyA signal region, a promoter sequence, and the above nucleic acid sequence encoding a desired protein is located between the termination codon and a polyA signal region of a gene that is expressed in a specific cell and/or tissue. Here, the above promoter sequence is preferably derived from a gene that is expressed in a specific cell and/or tissue. Furthermore, the polyA signal region to be located together with the above nucleic acid sequence encoding a desired protein may be same as or different from the polyA signal region of a gene that is expressed in a specific cell and/or tissue.

In still another embodiment, a sequence containing a promoter sequence, the above nucleic acid sequence encoding a desired protein, and a polyA signal region is located downstream of a polyA signal region of a gene that is expressed in a specific cell and/or tissue. Here, the above promoter sequence is preferably derived from a gene that is expressed in a specific cell and/or tissue. Furthermore, the polyA signal region to be located together with the above nucleic acid sequence encoding a desired protein may be the same as or different from the polyA signal region of a gene that is expressed in a specific cell and/or tissue. Furthermore, the distance between the polyA signal region of the gene that is expressed in a specific cell and/or tissue and the above promoter sequence is preferably less than 1 Kb.

In still another embodiment of the present invention, the pluripotent cell contains both a genome wherein the nucleic acid sequence encoding a desired protein is located so that the expression of the desired protein is regulated by the regulatory region of a gene that is expressed in at least a specific cell and/or tissue, and a genome wherein the allele of the gene that is expressed in the above specific cell and/or tissue is inactivated.

In another embodiment of the present invention, the pluripotent cell is an embryonic stem cell.

In still another embodiment of the present invention, the chimeric non-human animal is selected from the group consisting of mice, cattle, pigs, monkeys, rats, sheep, goats, rabbits, and hamsters. In a preferred embodiment of the present invention, the chimeric non-human animal is a mouse.

In still another embodiment of the present invention, a combination of a gene that is expressed in a specific cell and/or tissue and the specific cell and/or tissue deficient in a non-human animal strain is selected from the group consisting of the following (1) to (7):
(1) an immunoglobulin light chain or heavy chain gene and a B-lymphocyte;
(2) a T-cell receptor gene and a T-lyniphocyte;
(3) a myoglobin gene and a muscle cell;
(4) a crystallin gene and a crystalline lens of an eyeball;
(5) a renin gene and a kidney tissue;
(6) an albumin gene and a liver tissue; and
(7) a lipase gene and a pancreas tissue.

In a preferred embodiment of the present invention, the combination of a gene that is expressed in a specific cell and/or tissue and the specific cell and/or tissue deficient in a non-human animal strain is that of an immunoglobulin light chain κ gene and a B-lymphocyte.

In a second aspect, the present invention provides a method for producing a non-human animal expressing a desired protein, comprising obtaining an offspring capable of expressing the desired protein by crossing a chimeric non-human animal produced by the above method for producing a chimeric non-human animal.

Furthermore, in a third aspect, the present invention provides a chimeric non-human animal, which is derived from a pluripotent cell derived from a non-human animal containing a genome wherein a nucleic acid sequence encoding a desired protein is located so that the expression of the desired protein is regulated by the regulatory region of a gene that is expressed in a specific cell and/or tissue and a host embryo of a non-human animal strain deficient in the above specific cell and/or tissue, and is capable of expressing the above desired protein in at least the above specific cells and/or tissues.

In one embodiment of the present invention, the above nucleic acid sequence encoding a desired protein is located downstream of the regulatory region of a gene that is expressed in a specific cell and/or tissue. In another embodiment, the above nucleic acid sequence encoding a desired protein is located downstream of an internal ribosomal entry site located downstream of the termination codon of a gene that is expressed in a specific cell and/or tissue.

In still another embodiment, a sequence containing an internal ribosomal entry site and the above nucleic acid sequence encoding a desired protein is located between the termination codon and a polyA signal region of a gene that is expressed in a specific cell and/or tissue.

In still another embodiment, a sequence containing a polyA signal region, a promoter sequence, and the above nucleic acid sequence encoding a desired protein is located between the termination codon and a polyA signal region of a gene that is expressed in a specific cell and/or tissue. Here, the above promoter sequence is preferably derived from a gene that is expressed in a specific cell and/or tissue. Furthermore, the polyA signal region to be located together with the above nucleic acid sequence encoding a desired protein may be same as or different from the polyA signal region of a gene that is expressed in a specific cell and/or tissue.

In still another embodiment, a sequence containing a promoter sequence, the above nucleic acid sequence encoding a desired protein, and a polyA signal region is located downstream of a poly A signal region of a gene that is expressed in a specific cell and/or tissue. Here, the above promoter sequence is preferably derived from a gene that is expressed in a specific cell and/or tissue. Furthermore, the polyA signal region to be located together with the above nucleic acid sequence encoding a desired protein may be the same as or different from the polyA region of a gene that is expressed in a specific cell and/or tissue. Furthermore, the distance between the polyA signal region of a gene that is expressed in a specific cell and/or tissue and the above promoter sequence is preferably less than 1 Kb.

In still another embodiment of the present invention, the pluripotent cell contains both a genome wherein the nucleic acid sequence encoding a desired protein is located so that the expression of the desired protein is regulated by the regulatory region of a gene that is expressed in at least a specific cell and/or tissue, and a genome wherein the allele of the gene that is expressed in the above specific cell and/or tissue is inactivated.

In another embodiment of the present invention, the pluripotent cell is an embryonic stem cell.

In still another embodiment of the present invention, the chimeric non-human animal is selected from the group consisting of mice, cattle, pigs, monkeys, rats, sheep, goats, rabbits, and hamsters. In a preferred embodiment of the present invention, the animal is a mouse.

In still another embodiment of the present invention, a combination of a gene that is expressed in a specific cell and/or tissue and the specific cell and/or tissue deficient in a non-human animal strain is selected from the group consisting of the following (1) to (7):
(1) an immunoglobulin light chain or heavy chain gene and a B-lymphocyte;
(2) a T-cell receptor gene and a T-lymphocyte;
(3) a myoglobin gene and a muscle cell;
(4) a crystallin gene and a crystalline lens of an eyeball;
(5) a renin gene and a kidney tissue;
(6) an albumin gene and a liver tissue; and
(7) a lipase gene and a pancreas tissue.

In a preferred embodiment of the present invention, the combination of a gene that is expressed in a specific cell and/or tissue and the specific cell and/or tissue deficient in a non-human animal strain is that of an immunoglobulin light chain κ gene and a B-lymphocyte.

Furthermore, in a fourth aspect, the present invention provides a method for analyzing the *in vivo* functions of a desired protein or a gene encoding the desired protein, comprising comparing the phenotype of the above chimeric non-human animal or an offspring of the above chimeric non-human animal capable of expressing the desired protein with that of a corresponding wild-type non-human animal containing no nucleic acid sequence of a gene encoding the desired protein, so as to determine differences in these phenotypes.

Furthermore, in a fifth aspect, the present invention provides a tissue or cell derived from any of the above chimeric non-human animals. The cell or tissue contains a genome wherein a nucleic acid sequence encoding a desired protein is located so that the expression of the desired protein is regulated by the regulatory region of a gene that is expressed in the cell or the tissue and can express the desired protein.

In one embodiment of the present invention, the above tissue or cell is selected from the group consisting of B-lymphocytes, T-lymphocytes, muscle cells, crystalline lenses of eyeballs, kidney tissues, liver tissues, and pancreas tissues.

In a sixth aspect, the present invention also provides a hybridoma that is produced by the fusion of a cell derived from the above tissue or cell and a proliferable tumor cell.

Furthermore, in a seventh aspect, the present invention provides a method for producing a desired protein, comprising causing the production of the desired protein using any of the above chimeric non-human animals, the above tissues or cells, or the above hybridomas, and then collecting the proteins.

The terms relating to the present invention are as defined below.

The term "non-human animal" used in this specification means an animal excluding humans, and is generally selected from vertebrates comprising fish, reptiles, amphibians, birds, and mammals, and is preferably selected from mammals. In the production of chimeric non-human animals in the present invention, embryonic stem cells are preferably utilized as pluripotent cells. Thus, the non-human animals intended by the present invention encompass all non-human animals including non-human animals for which embryonic stem cells can be established (e.g., mice, cattle, sheep, pigs, hamsters, monkeys, goats, rabbits, and rats), and other non-human animals for which embryonic stem cells can be established in the future. The term "chimeric non-human animal" means an animal formed of both differentiated cells derived from a pluripotent cell (described later), and differentiated cells derived from a host embryo (Bradley et al., Nature, 309: 255-6, 1984). Experimentally, the birth of an animal (0% chimera) wherein all the cells are derived from a host embryo, or an animal (100% chimera) wherein all the cells are derived from a pluripotent cell is possible. Strictly speaking, these animals are not "chimera." However, the term "chimeric non-human animal" encompasses these animals for convenience.

The term "pluripotent cell" or "cell having pluripotency" used in this specification means, in the production of the above chimeric non-human animal, a cell that can differentiate into 2 or more types of cells or tissues of a chimeric non-human animal by injection into a host embryo or by formation of aggregate embryos. Specific examples of such pluripotent cells include embryonic stem cells (ES cells), embryonic germ cells (EG cells), and embryonal carcinoma cells (EC cells).

The term "embryonic stem cell" used in this specification is also referred to as an ES cell (Embryonic Stem cell), and means an early embryo-derived cultured cells characterized in that such cells can proliferate while maintaining anaplasticity (totipotency). Specifically, embryonic stem cells are of a cell line that is established by culturing cells of an inner cell mass that are undifferentiated stem cells, existing inside the blastocyst in an early embryo of an animal, so that the cells keep proliferating while maintaining their undifferentiated state. Furthermore, the term "embryonic germ cell" used in this specification is also referred to as an EG cell, and means a cultured cell derived from a primordial germ cell, which is characterized in that it has ability almost equivalent to that of the above embryonic stem cell. The embryonic germ cells are of a cell line that is established by culturing primordial germ cells obtained from an embryo several days to several weeks after fertilization (for example, in the case of a mouse, an approximately 8.5 days old embryo) so that the cells keep proliferating while maintaining their undifferentiated state.

The term "desired protein" used in this specification refers to a protein to be expressed is attempted in at least 1 type of cell and/or tissue of a chimeric non-human animal produced by the method of the present invention. The functions of such a protein may be either known or unknown. The above desired protein may be a functional secretory protein, a functional membrane protein, a functional intracellular or intranuclear protein, a soluble portion of a functional membrane protein having a secretory signal added thereto, or the like, which are derived from mammals. Here, the term "functional" means to have a specific type of work, action, or function *in vivo.*

In the case of a protein with known functions, new findings may be obtained about the interactive relationship of the functions of the protein by observing the effect of high level expression of the desired protein in at least one type of cell and/or tissue of a chimeric non-human animal. In the case of a protein with unknown functions, findings leading to the elucidation of the functions of the protein may also be obtained by observing the effect of the high level expression. The "desired protein" in the present invention will be expressed in a chimeric non-human animal into which the gene thereof has been introduced. The "desired protein" may be not expressed or is expressed poorly in a specific cell and/or tissue that is caused to express the protein according to the present invention, or it may refer to a protein derived from an heterologous animal. The types of the desired protein may be any proteins of interest.

The term "nucleic acid sequence encoding a desired protein" used in this specification may refer to either endogenous or exogenous DNA. Examples of the exogenous DNA include a human-derived DNA. In this specification, the term "a structural gene encoding a desired protein" is used as a synonym for the above term.

The term "expression" used with reference to a protein in this specification has a meaning equivalent to the same term used with reference to the expression of a gene encoding the protein.

The term "regulatory region" used in this specification refers to a general term such as "regulatory sequence," "control sequence," "control region," or the like, and indicates a region having functions to regulate or control gene expression (transcription, translation, or protein synthesis). Examples of such a regulatory region include, but are not limited to, a promoter, an enhancer, and a silencer. Furthermore, the term "regulatory region" in the present invention may refer to a region containing one functional element (e.g., one promoter sequence) or a region containing multiple elements (e.g., 1 promoter sequence, 1 enhancer sequence, and the like). Furthermore, the term "promoter sequence'' refers to one type of a regulatory region known by persons skilled in the art, and indicates a nucleotide sequence that is located upstream of a structural gene to which RNA polymerase binds upon the start of transcription.

The term "internal ribosomal entry site" used in this specification is abbreviated as IRES (Internal Ribosomal Entry Site), and is known as an element that enables polycistronic expression. An IRES indicates a site that forms a unique RNA secondary structure, and enables the initiation of translation by a ribosome from the initiation codon located downstream of the RNA secondary structure. In the case of mammals, an IRES is inferred to be involved in the event of the initiation of translation and protein synthesis by binding to the subunit for the decoding of a ribosome so as to cause a conformational change whereby an adjacent region encoding a protein is brought into the decoding site (Spahn et al., Science 291: 1959, 2001).

The term "poly A signal region" used in this specification indicates a nucleotide sequence portion that is located at the end portion of a transcription region and directs the addition of polyadenylic acid (A) chain to the 3' untranslated region of an mRNA precursor after transcription.

The terms "upstream" and "downstream" used in this specification indicates directions toward the 5' end and the 3' end, respectively, in a nucleic acid sequence such as a genome and a polynucleotide.

The terms "bp (base pair)" and "Kb (kilobase pair)" used in this specification indicate the length and distance of a nucleic acid sequence. 1 bp represents one base pair, and 1 Kb corresponds to 1000 bp.

The term "allele" used in this specification means a gene that is located in homologous regions of a homologous chromosome, and is also functionally homologous in an organism having a genome that is polyploid. Both alleles are generally expressed. Furthermore, the term "allelic exclusion" in terms of an individual organism refers to a situation where, although a trait derived from both alleles is represented, only either one of the alleles is randomly expressed in individual cells, and the expression of the other is excluded. For example, this is found in the gene of an antibody or a T cell receptor, and is due to the fact that only one complete gene is formed by the recombination of one variable region gene as a signal.

The term "soluble portion of a membrane protein having a secretory signal added thereto" used in this specification means an extracellular domain among membrane protein molecules, to which a secretory signal (or a signal sequence) has bound.

The term "immunoglobulin light chain gene" used in this specification indicates a gene encoding the light chain (or L chain) of an immunoglobulin (Ig) molecule. The light chain includes the κ chain and the λ chain, and is composed of a variable (V) region and a constant (C) region. Moreover, a light chain gene is composed of one C region gene, multiple V region genes, and multiple junction (J) region genes.

The term "host embryo of a non-human animal strain deficient in a specific cell and/or tissue" or "deficient host embryo" used in this specification refers to a host non-human animal embryo for injecting a pluripotent cell, and means an embryo deficient in the cell and/or tissue.

The term "offspring" used with reference to the chimeric non-human animal in this specification refers to an offspring obtained by the crossing of chimeric non-human animals of the present invention, or a chimeric non-human animal of the present invention with a non-human animal of the same species, and means a non-human animal expressing a desired protein in at least a specific cell and/or tissue.

The term "phenotype" used in this specification indicates an original trait of an animal, or a trait of an animal that appears as a result of the presence a transgene.

The term "tumor cell capable of proliferation" used in this specification means a cell capable of permanently proliferating by tumorigenesis. Examples of such a tumor cell include cells of plasmocytoma (e.g., myeloma cells) that produce immunoglobulins.

The term "hybridoma" used in this specification indicates a hybrid cell that is formed by fusing a cell derived from a tissue or a cell obtained from the chimeric non-human animal of the present invention or an offspring thereof with the above tumor cell capable of proliferation.

The term "knock-in vector" used in this specification indicates a vector that is used for the expression of a desired protein by introducing a gene encoding the desired protein to a target gene locus by homologous recombination. In addition, the term "knock-in" or "gene knock-in" refers to the introduction of a nucleic acid sequence encoding a desired protein to a target gene locus by homologous recombination, thereby causing the expression of the desired protein.

The term "knock-out vector" used in this specification indicates a vector for disrupting or inactivating a target gene of a non-human animal by homologous recombination. Moreover, the term "knock-out" or "gene knock-out" means the introduction of a structure that inhibits the expression of a gene to the target gene locus by homologous recombination, thereby disrupting or inactivating the target gene.

The term "targeting vector" used in this specification indicates a vector that can be introduced by homologous recombination to a target position in the genome of a non-human animal. The term "targeting vector" encompasses the above "knock-in vector" and "knock-out vector." In addition, the term "targeting" or " gene targeting" means to introduce by homologous recombination a desired gene structure to a target position in the genome of a non-human animal.

The term "polyamine" used in this specification is a general term referring to a linear aliphatic hydrocarbon having 2 or more primary amino groups.

The present invention will be described in detail as follows. This application claims a priority from Japanese Patent Application No. 2002-42321 filed February 19, 2002, which claims a priority from Japanese Patent Application No. 2001-350481 filed November 15, 2001. This application includes content disclosed in the specifications and/or drawings of the above Japanese Patent Applications.

The present inventors have developed a method for producing a chimeric non-human animal expressing a desired protein at high levels in at least a specific cell and/or tissue without being largely affected by chimerism by applying the above BC method. That is, the method for producing a chimeric non-human animal of the present invention comprises the steps of:
1) preparing a pluripotent cell derived from a non-human animal that contains a genome wherein a nucleic acid sequence encoding a desired protein is located so that the expression of the desired protein is regulated by the regulatory region of a gene that is expressed in at least a specific cell and/or tissue;
2) obtaining a chimeric embryo by injecting the pluripotent cell prepared in the above step 1 into a host embryo of a non-human animal strain deficient in the above specific cell and/or tissue;
3) transplanting the chimeric embryo obtained in the above step 2 into a foster parent non-human animal of the same species; and
4) selecting a chimeric non-human animal expressing the desired protein in at least the above specific cell and/or tissue from among offsprings obtained after the transplantation in the above step 3.

In the method of the present invention, the deficient cell and/or tissue in a host embryo is complemented by the above pluripotent cell by blastocyst complementation (BC) as described above. Specifically, regardless of the chimerism of the produced whole chimeric non-human animal, all the cells and/or tissues are derived from the pluripotent cell. As a result, as long as the pluripotent cell carries a gene (nucleic acid sequence) encoding a desired protein so that the gene is regulated by the regulatory sequence of a gene that is expressed in the above cell and/or tissue, high level expression of the desired protein in the cell and/or tissue of a chimeric non-human animal can be expected.

### 1. Preparation of pluripotent cells

In the method for producing a chimeric non-human animal of the present invention, first, a pluripotent cell derived from a non-human animal is prepared, such cell containing a genome wherein a nucleic acid sequence (structural gene) encoding a desired protein is located (inserted). The nucleic acid sequence is located so that the expression of the desired protein is regulated by the regulatory region of a gene that is expressed in a specific cell and/or tissue.

As the cell having pluripotency in the present invention, those as defined above can be utilized. Embryonic stem cells (ES cells) are preferred, and particularly, mouse ES cells are preferred.

In addition, a gene that is expressed in a specific cell and/or tissue may be expressed tissue-specifically or constitutively. Examples of a gene that is expressed tissue-specifically include an immunoglobulin light chain or heavy chain gene, a T cell receptor gene, a myoglobin gene, a crystallin gene, a renin gene, a lipase gene, and an albumin gene. Furthermore, an example of a gene that is constitutively expressed is a hypoxanthine guanine phosphoribosyl transferase (HPRT) gene. When the gene is a gene that is expressed tissue-specifically in a chimeric non-human animal, an embryo of a strain deficient in a cell and/or a tissue wherein the gene is expressed is employed as a host embryo described later. In the case of a gene that is constitutively expressed, an embryo of a strain deficient in any cell and/or tissue is employed as a host embryo described later.

A nucleic acid sequence (structural gene) encoding a desired protein should be located (ligated or inserted) in a way that the expression of the desired protein is regulated by the regulatory region of a gene that is expressed in at least a specific cell and/or tissue. Hence, the nucleic acid sequence is located downstream of the regulatory region of a gene that is expressed in a specific cell and/or tissue.

Alternatively, the nucleic acid sequence encoding the desired protein is located downstream of the internal ribosomal entry site (IRES), that is located between the termination codon and a sequence encoding a polyA signal region of a gene that is expressed in a specific cell and/or tissue. Specifically, the nucleic acid sequence is in a state of being functionally ligated to the IRES on the genome, and is present between the termination codon and a sequence encoding a polyA signal region of a gene that is expressed in a specific cell and/or tissue. Upon construction of a knoek-in vector, the polyA signal region of a gene that is expressed in the above specific cell and/or tissue can be used, but other polyA signal regions can be used as well. For example, other polyA sequences known in the art such as a polyA signal region derived from simian virus 40 (SV40) can also be used.

The nucleic acid sequence encoding a desired protein is located between the termination codon and a sequence encoding a polyA signal region of a gene that is expressed in a specific cell and/or tissue, and the sequence is inserted in the form that the sequence is located downstream of a promoter sequence that is located downstream of a sequence encoding a 2nd polyA signal region. Specifically, the nucleic acid sequence is present in a state of being functionally ligated to the promoter sequence and the sequence encoding the polyA signal region on the genome, and the gene that is expressed in a specific cell and/or tissue originally existing on the genome is also present in a state of being functionally ligated to the promoter sequence and the sequence encoding the polyA signal region. Upon construction of a knock-in vector, examples of a promoter sequence used herein are not specifically limited, as long as they are capable of regulating the expression in a specific cell and/or tissue. Preferably, the promoter of a gene that is expressed in a specific cell and/or tissue is used. When 2 promoters are present in a knock-in vector, the two promoters may be the same or differ from each other, as long as they are capable of regulating the expression in the same cell and/or tissue. Furthermore, examples of a sequence encoding a polyA signal region used for the construction of a knock-in vector are not specifically limited, as long as the polyA signal region is known in the art, and include a polyA signal region derived from the same origin as that of a promoter and a polyA signal region derived from simian virus 40 (SV40). Furthermore, similarly to the case of a promoter, when sequences encoding 2 polyA signal regions are present in a knock-in vector, the two may be the same or differ from each other.

Moreover, the above nucleic acid sequence encoding a desired protein can also be located in the order of a promoter sequence, the nucleic acid sequence, and a sequence encoding a polyA signal region downstream of the polyA signal region of a gene that is expressed in a specific cell and/or tissue. Specifically the nucleic acid sequence is present in a state (cassette form) of being functionally ligated to a promoter and a sequence encoding a polyA signal region, downstream of the polyA signal of a gene that is expressed in a specific cell and/or tissue. With reference to construction of a knock-in vector, examples of a promoter sequence used herein are not specifically limited, as long as they are capable of regulating the expression in a specific cell and/or tissue. Preferably, the promoter of a gene that is expressed in a specific cell and/or tissue is used. Furthermore, with reference to construction of a knock-in vector, examples of a sequence encoding a polyA signal region are not specifically limited, as long as the polyA signal region is known in the art, and include a polyA signal region derived from the same origin as that of a promoter and a polyA signal region derived from simian virus 40 (SV40). In addition, when sequences encoding 2 poly A, signal regions are present in a knock-in vector, the two sequences may be the same or differ from each other. The distance between the 3' end of the polyA signal region of a gene that is expressed in a specific cell and/or tissue and the 5' end of a promoter sequence that regulates the expression of a nucleic acid sequence encoding a desired protein is not specifically limited, as long as the nucleic acid sequence can be expressed in a specific cell and/or tissue. The longer distance thereof may provide an unfavorable effect on the stability of mRNA, the transcription product. In addition, this also leads to a larger structure for a targeting vector, making it difficult to prepare a vector with such a structure. Because of these reasons, the distance between the 3' end of the polyA signal region and the 5' end of the promoter sequence regulating the expression of a nucleic acid sequence encoding a desired protein is preferably within 1 Kb.

When a nucleic acid sequence (structural gene) encoding a desired protein is located downstream of the above regulatory region, the nucleic acid sequence may be inserted downstream of the regulatory region. Alternatively, for an allele on the one side of an ES cell, the nucleic acid sequence can also be simply located in a form whereby the sequence is located immediately following the regulatory sequence of a gene that is expressed in a cell and/or tissue, so that the gene is substituted with an original structural gene. The original structural gene that has been substituted with the nucleic acid sequence encoding the desired protein is expressed by an allele on the other side, so that the cell and/or tissue can maintain its normal conditions. However, in a gene such as an immunoglobulin gene wherein allelic exclusion works, an IRES sequence is located following the termination codon of the original structural gene, and then the nucleic acid sequence encoding a desired protein can be located following the IRES sequence in alleles on both sides of an ES cell. Moreover, an allele on one side of an original structural gene is previously inactivated in an ES cell, an IRES sequence is located following the termination codon of an original structural gene of an allele that has not been inactivated, and then the nucleic acid sequence encoding a desired protein can also be located following the IRES sequence. In this case, the allele that has not been inactivated is expressed exclusively, so that high-level expression of the nucleic acid sequence encoding a desired protein is expected at the same time.

Expression of an immunoglobulin κ chain gene occurs by the binding of many V and J gene fragments by recombination as described above. As a result of this binding, a promoter sequence existing in the vicinity of the upstream of each V gene fragment is located in the vicinity of an enhancer sequence that is present downstream of the J fragment. An enhancer sequence can only activate the promoter in such a situation (Picard et al., Nature, 307: 80-2, 1984). Specifically, the above nucleic acid sequence encoding a desired protein can also be located in the vicinity of the enhancer sequence, while being artificially ligated to the promoter sequence of an immunoglobulin κ chain gene. In the immunoglobulin κ chain gene locus, another enhancer sequence is known to be present downstream of the above enhancer sequence (Meyer et al., EMBO J. 8: 1959-64, 1989). This gene is expressed at high levels in B cells under the influence of such multiple enhancers.

A pluripotent cell derived from a non-human animal comprising a genome wherein the above-mentioned nucleic acid sequence encoding a desired protein is located is hereinafter referred to as a knock-in cell or a knock-in ES cell. For example, these cells can be obtained as described below, but a method for obtaining the cells is not limited thereto.

### (1) Construction of a targeting vector (knock-in vector)

A knock-in vector wherein a nucleic acid sequence encoding a desired protein has been inserted downstream of a gene that is expressed in a specific cell and/or tissue or a knock-in vector wherein a nucleic acid sequence encoding a desired protein is inserted downstream of an internal ribosomal entry site that has been located downstream of a gene that is expressed in a specific cell and/or tissue are constructed.

A nucleic acid sequence to be introduced may be a cDNA or a genomic DNA containing introns, as long as it contains a region from the initiation codon to the termination codon. A protein encoded by the nucleic acid sequence may be of any type. The nucleic acid sequence used in the present invention may be used for high-level expression or secretion of the protein encoded by the sequence, or may also be used for elucidating the functions of the protein. Therefore, any type of nucleic acid sequence to be introduced can be used, as long as the nucleotide sequence thereof is specified. Examples of a nucleic acid sequence (structural gene) include genes encoding functional proteins derived from mammals, and preferably, humans, such as a gene encoding a secretory protein, a gene encoding a membrane protein, and a gene encoding intracellular or intranuclear protein.

An example of an IRES that can be utilized in the present invention is, but is not limited thereto, an IRES derived from encephalomyocarditis virus (EMCV) (Jang *et al., supra*), pIREShyg (Clontech, U.S.A.). When an IRES and/or a nucleic acid sequence encoding a desired protein is inserted into a knock-in vector, an insertion site therefor is preferably located between the termination codon and a polyA addition site of a gene that is expressed in a specific cell and/or tissue. In addition, a plural number thereof may be inserted. To prevent the secondary structure formed by an IRES sequence from having an adverse effect on the translation of the above gene, it is preferable to provide a certain space (e.g., several bp to several 10 bp) between the termination codon gene of the above and the IRES.

To alter the genome of an animal so that it contains a nucleic acid sequence encoding a desired protein downstream of a gene that is expressed in a specific cell and/or tissue, or so that is contains an IRES downstream of a gene that is expressed in a specific cell and/or tissue, and a nucleic acid sequence encoding the desired protein downstream of the IRES, a knock-in vector is prepared. To this vector DNA, the IRES and/or the nucleic acid sequence encoding a desired protein is inserted. Examples of a vector that can be used for this purpose include plasmids and viruses. Persons skilled in the art can easily select and obtain a vector that can be used as a knock-in vector. A specific example of a vector is, but is not limited to, pKIκ (see examples described later). In the knock-in vector, between the termination codon and a polyA addition site (e.g., around the halfway point) of a gene that is expressed in a specific cell and/or tissue, an appropriate restriction enzyme cleavage site is inserted as an insertion site for an IRES sequence and/or a target nucleic acid sequence DNA. In the restriction enzyme cleavage site, a DNA (cDNA or genomic DNA) containing a region from the initiation codon to the termination codon of a nucleic acid sequence to be introduced is inserted. Furthermore, preferably, a translational stimulatory sequence such as a Kozak sequence can be located upstream of the initiation codon. Furthermore, the vector can contain a selection marker, for example, a puromycin-resistance gene, a neomycin-resistance gene, a blasticidin-resistance gene, or a GFP gene, if necessary.

### (2) Introduction of knock-in vector into pluripotent cell derived from non-human animal, and selection of homologous recombinant

Pluripotent cells derived from a non-human animal can be transformed with a knock-in vector according to a known technique in the art, for example, the method described in Bio Manual Series 8, Gene Targeting, Shinichi Aizawa, YODOSHA, 1995. For example, the above-prepared knock-in vector can be introduced into pluripotent cells by electroporation, the lipofection method, or the like.

In the step of preparing a knock-in (targeting) vector DNA, the ratio of homologous recombination to random insertion can be improved by treating targeting vector DNAs with polyamines or analogues thereof, or forming complexes of targeting vector DNAs and polyamines or analogues thereof. Such treatment with polyamines or analogues thereof, or the formation of complexes with polyamines or analogues thereof, can be performed, before transformation, or before, during, or after the linearization of the targeting vector, by bringing the vector into contact with at least one type of polyamine or an analogue thereof. For example, an increase in the ratio of homologous recombination is observed by adding 1 mM spermidine to a reaction buffer used when a knock-in vector is linearized.

The timing for bringing targeting vectors into contact with polyamines or analogues thereof may be as described above in accordance with any of the following cases: polyamines or analogues thereof are added into a solution containing the vector before linearization of the vector; polyamines or analogues thereof are added into a restriction enzyme buffer for the linearization of the vector (e.g., 50 mM Tris-HCl, 10 mM MgCl₂, 100 mM NaCl, 1 mM Dithioerythritol); and/or polyamines or analogues thereof are added to an HBS buffer (e.g., 25 mM Hepes, 137 mM NaCl, 5 mM KCl, 0.7 mM, Na₂HPO₄·2H₂O, 6 mM Dextrose) for dissolving DNA after restriction enzyme treatment, phenol/chloroform treatment, ethanol precipitation, and dry treatment. In any of these cases, in an electric pulse experiment, DNA to be added to cells is preferably in a state of forming a complex with a polyamine or an analogue thereof. The concentration for addition of polyamines or analogues thereof is preferably between 0.1 mM and 10 mM, and is further preferably 1 mM. Addition at a concentration of 1 mM is used in a preferred embodiment of this invention. At a lower or a higher concentration than 1 mM, a similar effect can be exhibited.

As representative polyamines other than spermidine (triamine), for example, putrescine (diamine), cadaverine (diamine), and spermine (tetraamine) are known. These polyamines are known to have physiological action similar to that of spermidine, specifically, 1) stabilization and action causing conformational changes in a nucleic acid by interaction with the nucleic acid, 2) promoting action of various nucleic acid synthesis systems, 3) activation of protein synthesis, and 4) acetylation of histone, and the like. These polyamines are thought to exhibit effects analogous to that of spermidine. Known important features of polyamines are to form a complex with DNA, and to be a promoting factor in an *in vitro* recombination experimental system using an enzyme derived from prokaryotic cells. Furthermore, it is also known that by the addition of polyamines to foreign DNA, the efficiency of the introduction of the DNA into a mammalian cell is improved. In the meantime, polyamines have not conventionally been known to have an effect on the ratio of homologous recombinants to clones with random insertion that is an object of the present invention. The above polyamines are commercially available. For example, spermidine (manufactured by SIGMA, U.S.A.) is available. In addition, analogues of polyamines are not specifically limited, as long as they are compounds that can ionically bind to the phosphoric acids of DNA in a manner similar to that of polyamines. For example, poly-L-lysine and poly-L-arginine are known. It is thought that these analogues also provide stabilization and conformational changes in DNA by ionically binding to phosphoric acids of DNA, thereby exhibiting an effect similar to that provided by polyamines. Commercially available analogues can be used as the above analogues. For example, poly-L-lysine and poly-L-arginine (manufactured by SIGMA, U.S.A.) are available.

As described above, by the utilization of polyamines or analogues thereof, the ratio of homologous recombination to random insertion can be improved. This use is thought to also have an effect on gene disruption (gene knock-out) via homologous recombination. That is, the present invention provides a method for gene targeting that comprises treating a targeting vector (knock-in vector or knock-out vector) with polyamines or analogues thereof, or a method for gene targeting that comprises forming complexes of targeting vectors and polymines or analogues thereof.

The present invention further provides a reagent or a composition comprising at least 1 type of polyamine or analogue thereof to be used for the gene targeting method. Examples of such polyamines or analogues thereof include, but are not limited to, spermidine, cadaverine, putrescine, spermine, poly-L-lysine, and poly-L-arginine. A preferred example is spermidine. When pluripotent cells derived from a non-human animal are transformed according to the present invention using a targeting vector treated with polyamines or analogues thereof as described above, or a targeting vector that forms complexes with polyamines or analogues thereof, the ratio of homologous recombinants to clones with random insertion can be improved compared with conventional cases (see Example 11).

Furthermore, the structure of a targeting vector (knock-in vector or knock-out vector) can be altered to increase the efficiency of homologous recombination. Specifically, the efficiency of homologous recombination can be increased by processing the targeting vector so as to prevent a negative selection marker used for eliminating a cell having a targeting vector randomly inserted in the genome from being exposed at the end portion of the vector when the targeting vector is linearized.

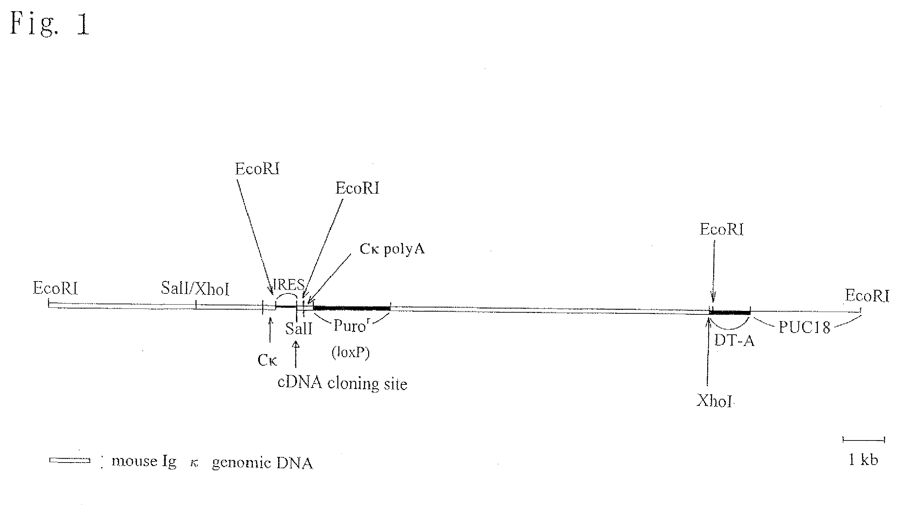

Specifically, in the linearized targeting vector, it is preferred to process so that the 5' end and the 3' end of a gene structure functioning as a negative selection marker are located at least 1 Kb, and preferably 2 Kb or more, away from the 5' end and 3' ends of the targeting vector, respectively. In general, since a region for homologous recombination with a genome (homologous recombination region) is located on either the 5' or the 3' end of a negative selection marker, the distance from the vector end is 3 Kb or more in most cases. Furthermore, the other end of the negative selection marker is adjacent to the end of the vector in most cases. In the present invention, one end of the negative selection marker, to which no homologous recombination region is adjacent, is processed to provide a distance of at least 1 kb from the terminus of a linearized vector, thereby increasing the efficiency of homologous recombination. As a sequence to secure the distance from the vector end, a plasmid sequence such as pUC used for the construction of a targeting vector can be kept intact (that is, so that this sequence is not deleted upon linearization) and then utilized (e.g., see Figs. 1 to 7). Alternatively, any new non-coding sequence that is not homologous to a targeting region of interest can be located adjacent to the negative selection marker. When linearization of a vector is carried out, restriction enzyme recognition sites of a targeting vector used in this case are thoroughly examined to select an appropriate restriction enzyme site with which a distance between the vector end and the end of the negative selection marker can be secured. Then the vector is linearized, so that an effect of improving the efficiency of homologous recombination can be achieved. In addition, when such an appropriate restriction enzyme site cannot be found, an appropriate restriction enzyme recognition sequence can be introduced at a desired position in the targeting vector by a technique using PCR (Akiyama et al., Nucleic Acids Research, 2000, Vol. 28, No.16, E77.).

It is thought that the use of the above-described targeting vector structure results in decreased frequency of attacks on the negative selection marker within cells by nuclease, and increases the efficiency of homologous recombination.

Specifically, the present invention provides a gene targeting vector wherein the 5' end and the 3' end of a gene structure functioning as a negative selection marker are located at least 1 Kb, and preferably 3 Kb or more, away from the 5' end and the 3' end, respectively, of the linearized targeting vector, and a method for gene targeting, which uses the targeting vector. In the above targeting vector, as a negative selection marker, any known marker can be utilized. A preferred negative selection marker is a diphtheria toxin A gene.

To conveniently identify homologous recombinants, a drug resistance gene marker can be previously inserted into a position, at which a foreign gene is knocked in. For example, TT2F mouse ES cells used in the examples of this specification are derived from F1 individuals obtained by the crossing of a C57BL/6 strain with a CBA strain. As described above (Deng & Capecchi, Mol. Cell. Biol., 12: 3365-71, 1992), when a sequence of a genomic homologous region contained in a knock-in vector is derived from C57BL/6, it is predicted that homologous recombination will occur at a higher rate in an allele derived from C57BL/6 in TT2F cells. Specifically, from the start, a targeting vector (knock-in vector) containing C57BL/6-derived genomic DNA is used, and, for example, a G418-resistance marker can be inserted into a C57BL/6-derived allele. Next, a knock-in vector containing a puromycin-resistance marker and the C57BL/6-derived genomic DNA is introduced into the obtained G418-resistant strain, so that a puromycin-resistant and G418-sensitive strain can be obtained. In this strain, the G418-resistance gene has been removed by homologous recombination of the knock-in vector with the gene that is expressed in the above specific cell and/or tissue, and instead, a structural gene encoding a desired protein and the puromycin resistance marker have been inserted. With such a method, the trouble of Southern analysis or the like in identification of homologous recombinants can be avoided.

In a manner similar to the method described in PCT international application WO 00/10383 pamphlet filed by this applicant (international publication, March 2, 2000), puromycin-resistant clones are picked up, genomic DNA is prepared, and then homologous recombinants can be identified by the Southern analysis method. The puromycin-resistance gene in the knock-in vector is derived from a Lox-P Puro plasmid described in the WO 00/10383 pamphlet, and contains in forward direction Lox-P sequences on both ends. Thus, by the method described in the WO 00/10383 pamphlet, this resistance gene can be removed from pluripotent cells into which the gene has been knocked-in.

The above-mentioned knock-in vector, and techniques and means for improving the efficiency of homologous recombination, can be applied for all the cells into which a gene can be Introduced, and the use thereof is not limited to the production of chimeric animals. For example, in gene therapy directed to humans and human cells (e.g., blood cells and immunocytes), the knock-in vector and the technique for improving the efficiency of homologous recombination described in this specification can be used for disrupting or introducing a desired gene.

### 2. Host embryo deficient in specific cell and/or tissue

Next, in the method for producing chimeric non-human animals of the present invention, a host embryo (hereinafter also referred to as a deficient host embryo) of non-human animal strain which is deficient in a specific cell and/or tissue is prepared. Examples of such a deficient host embryo include, when an immunoglobulin light chain gene is utilized as a regulatory region, an embryo deficient in B-cells due to the knock-out of an immunoglobulin heavy chain gene (Tomizuka et al., Proc. Natl. Acad. Sci. U.S.A., 18: 722-727, 2000); when a T cell receptor gene is utilized as a regulatory region, an embryo deficient in T-lymphocytes due to deficiency in the T cell receptor β chain (Mombaerts et al., Nature, 360: 225-227, 1992); when a myoglobin gene is utilized as a regulatory region, an embryo deficient in muscle tissue due to the knock-out of a myogenin gene (Nabeshima et al., Nature, 364: 532-535, 1993); when a crystallin gene is used as a regulatory region, an embryo derived from an aphakia (ak) strain that is a mutant of a mouse deficient in lens (Liegeois et al., Proc. Natl. Acad. Sci. U.S.A., 93: 1303-1307, 1996), when a renin gene is utilized as a regulatory region, an embryo deficient in kidney tissue due to the knock-out of Sall1 gene (Nishinakamura et al., Development, 128: 3105-3115, 2001); when an albumin gene is utilized as a regulatory region, an embryo deficient in liver tissue due to c-Met gene deficiency (Bladt et al., Nature, 376: 768-770, 1995); and when a lipase gene is used as a regulatory region, an embryo deficient in pancreas tissue due to the knock-out of a Pdx1 gene (Jonsson et al., Nature, 371: 606-9, 1994). Preferred deficient host embryos are as exemplified above, but deficient host embryos that can be used in the present invention are not limited to the above embryos.

Furthermore, for the selection of the time for development, genetic backgrounds, and the like of host embryos in order to efficiently produce chimeric non-human animals, it is desired to use conditions that have been previously examined for each ES cell line. For example, in the case of a mouse, when a chimera is produced from TT2 cells or TT2F cells (wild-type color, Yagi et al., Analytical Biochemistry, 214: 70-76, 1993) derived from CBA × C57BL/6 F1, the genetic background of a host embryo is preferably Balb/c (white, CLEA JAPAN, INC., Japan), ICR (white, CELA JAPAN, INC., Japan) or MCH (ICR) (white, CLEA JAPAN, INC., Japan). Hence, it is desired to use an embryo (e.g., an 8-cell-stage embryo) of a non-human animal obtained by back-crossing of the above non-human animal strains deficient in specific cells/and or tissues with these strains as a deficient host embryo.

The deficient cell and/or tissue in a host embryo is complemented by pluripotent cells by blastocyst complementation (BC). Thus, the above deficient host embryo may be embryonic lethal, as long as it can develop into the blastocyst stage for the production of chimeric animals. Such an embryonic-lethal embryo is produced principally at a probability of one-fourth by crossing of animals having a gene deficiency heterologously. Hence, by obtaining a plural number of embryos by crossing, chimeric animals are produced according to the following procedures, and then animals wherein host embryos are deficient embryos are selected therefrom. This selection can be conducted by Southern analysis, PCR analysis, or the like using DNA extracted from the body tissues of the chimeric animals.

### 3. Production of chimeric embryo and transplantation of the embryo into foster parent

Chimeric non-human animals can be produced from the knock-in (ES) cell lines obtained in the above section "1. Preparation of pluripotent cells" by the method described by Shinichi Aizawa (*supra*) or the like. Specifically, the prepared knock-in pluripotent cells are injected into the blastocysts or 8-cell-stage embryos of the deficient host embryos described in the above section "2. Host embryo deficient in specific cell and/or tissue" using a capillary or the like. This embryonic blastocyst or the 8-cell-stage embryo is directly transplanted into the oviduct of a non-human animal that is a foster animal of the same species, or cultured for 1 day for the embryo to develop to a blastocyst, with the blastocyst then being transplanted into the uterus of the foster parent. Subsequently, the foster parents are fed to give birth, thereby obtaining offsprings.

### 4. Expression of transgene in chimeric non-human animal

The contribution ratio of the pluripotent cells in the offsprings derived from the knock-in pluripotent cell-injected embryos produced according to the above section "Production of chimeric embryo and transplantation of the embryo into foster parent" can be roughly determined based on coat color. For example, when the knock-in cell line derived from TT2F cells (wild color) is injected into a host mouse embryo whose background is MCH(ICR) (white), the proportion of wild color (dark brown) to the others represents the contribution ratio of the pluripotent cells. Here the contribution ratio determined by coat color is correlated with the contribution ratio of knock-in pluripotent cells in cells and/or tissues other than the above deficient cell and/or tissue. However, in some tissues, the contribution ratio of knock-in pluripotent cells may not agree with the contribution ratio determined by coat color. At the same time, in the above chimeric non-human animal, the above deficient cell and/or tissue derived from the host embryos is absent, and only those derived from the knock-in pluripotent cells are present. Recovery of the deficient cell and/or tissue in the chimeric non-human animal by the contribution of the knock-in pluripotent cells can be detected by FACS analysis (Fluorescence-Activated Cell Sorter), the ELISA method (Enzyme-Linked ImmunoSorbent Assay), or the like. Whether or not an inserted nucleic acid sequence (structural gene) is expressed in a cell and/or tissue that is derived from the knock-in pluripotent cell is detected by the RT-PCR method using RNA derived from the cell and/or tissue (Kawasaki et al., P.N.A.S., 85: 5698-5702, 1988), the Northern blot method (Ausubel et al., Current protocols in molecular biology, John Wiley & Sons, Inc., 1994), or the like. Expression at the protein level can be detected utilizing the enzyme immunoassay (ELISA; Toyama/Ando, "Tan-kurohn Koutai Jikken Manual (Monoclonal Antibody Experimental Manual)," Kodansha Scientific, 1987), the Western blot method (Ausubel *et al., supra*), or the like using chimeric mouse serum, when a specific antibody against a desired protein encoded by an introduced nucleic acid sequence is already present. Moreover, by the previous appropriate alteration of DNA encoding a nucleic acid sequence (structural gene) to be introduced, and addition of a tag peptide that is detectable with an antibody to the DNA, the expression of the introduced gene can be detected using the antibody or the like against the tag peptide (POD-labeled anti-His₆, Roche Diagnostics, Japan).

The chimeric non-human animal produced as described above expresses the introduced nucleic acid sequence (structural gene) at high levels in at least a specific cell and/or tissue. The thus expressed desired protein is of a system secreting blood, milk, or the like, this system can be utilized as a production system of a useful protein. Furthermore, when a protein with unknown functions is expressed at high levels, the functions of the protein can be elucidated based on the findings obtained upon the above high level expression.

In recent years, the combination of the method for producing animals from somatic-cell-nucleus-transferred embryos and gene targeting in somatic cells has further enabled gene alteration in animal species (e.g., cattle, sheep, and pigs) other than mice in a manner similar to that in the case of mice (McCreath et al., Nature, 405: 1066-1069, 2000). For example, cattle deficient in B cells can be produced by knock-out of immunoglobulin heavy chain. Moreover, a transgene is inserted downstream of the Ig gene locus of a mouse, cattle, sheep, pig or the like, an unfertilized egg is caused to develop wherein a nucleus of a fibroblast has been transferred, and then an ES cell can be prepared from an embryo at the blastocyst stage. A chimeric non-human animal can be produced using the ES cell and the above B-cell-deficient host embryo (Cibelli et al., Nature Biotechnol., 16: 642-646, 1998). Not only in mice, but also in other animal species, high level expression of a secretory protein is possible utilizing a similar expression system. By the use of large animals, this technique can be applied not only for the analysis of gene functions, but also for the production of a useful substance.

### 5. Production of offspring of chimeric non-human animal

The method for producing chimeric non-human animals of the present invention further comprises obtaining by the selection of a transgenic (Tg) animal having heterologously a nucleic acid sequence that has been introduced by crossing the chimeric non-human animal with a non-human animal of the same species, and obtaining an offspring of the Tg animal having the transgene homologously by crossing the male Tg animal with the female Tg animal.

### 6. Tissue or cell derived from chimeric non-human animal or an offspring thereof

In the present invention, tissues or cells derived from any of the above chimeric non-human animal or the offspring thereof can be obtained. The cell or tissue contains a genome wherein a nucleic acid sequence encoding a desired protein is located so that the expression of the desired protein is regulated by the regulatory region of a gene that is expressed in the cell or tissue, and can express the desired protein.

Examples of the above tissue and cell include any tissue and cell, such as B cells, the spleen, and the lymphoid tissue, as long as they are derived from a chimeric non-human animal or an offspring thereof, and can express a desired protein.

The above tissue and cell can be collected and cultured by techniques known in the art. Whether or not the tissue and cell express a desired protein can also be confirmed according to a routine technique. Such tissues and cells are useful in preparation of a hybridoma and in production of a protein, as described below.

### 7. Preparation of hybridoma

In the present invention, a hybridoma can be obtained by fusing a cell of a chimeric non-human animal expressing a nucleic acid sequence encoding an introduced desired protein in particular, a B cell or a cell obtained from the spleen containing B cells or from the lymphoid tissue with a tumor cell capable of proliferation (e.g., myeloma cell). A method for preparing a hybridoma can be based on, for example, a technique described in Ando, Chiba, "Tan-kurohn Koutai Jikken Sousa Nyuumon (Monoclonal Antibody Experimentation and Manipulation Introduction)," Kodansha Scientific, 1991.

As a myeloma, a cell incapable of producing an autoantibody derived from a mammal, such as a mouse, a rat, a guinea pig, a hamster, a rabbit, or a human can be used. In general, an established cell line obtained from mice, for example, 8-azaguanine-resistant mouse (derived from BALB/c) myeloma cell lines P3X63Ag8U.1 (P3-U1) [Yelton, D.E. et al., Current Topics in Microbiology and Immunology, 81: 1-7 (1978)], P3/NSI/1-Ag4-1(NS-1) [Kohler, G. et al., European J. Immunology, 6: 511-519 (1976)], Sp2/O-Ag14(SP-2) [Shulman, M. et al., Nature, 276: 269-270 (1978)], P3X63Ag8.653(653) [Kearney, J. F. et al., J. Immunology, 123: 1548-1550 (1979)], P3X63Ag8(X63) [Horibata, K. and Harris, A. W., Nature, 256: 495-497 (1975)] and the like are preferably used. These cell lines are subcultured in an appropriate medium, for example, a 8-azaguanine medium [the medium is prepared by adding 8-azaguanine to a RPMI-1640 medium supplemented with glutamine, 2-mercaptoethanol, gentamycin, and fetal calf serum (hereinafter referred to as "FCS")], Iscove's Modified Dulbecco's Medium (hereinafter referred to as "IMDM"), or Dulbecco's Modified Eagle Medium (hereinafter referred to as "DMEM"). 3 to 4 days before cell fusion, the cell lines are subcultured in a normal medium (e.g., DMEM medium containing 10% PCS), and 2x10⁷ or more cells are secured on the day of fusion.

Cells that can be used for the expression of a desired protein encoded by a nucleic acid sequence introduced within the cell are plasma cells (that is, plasmacytes) and their precursor cells, the lymphocytes. These cells may be obtained from any site of an individual, and can be generally obtained from the spleen, the lymph node, the bone marrow, the tonsil, peripheral blood, or an appropriate combination thereof. Splenocytes are most generally used.

Currently, the most generally conducted means for fusing a splenocyte expressing a desired protein encoded by an introduced nucleic acid sequence with a myeloma is a method using polyethylene glycol, which has relatively low cytotoxicity and with which the procedure for cell fusion is simple. Specifically, this can be conducted as follows. Splenocytes and myelomas are washed well in a serum-free medium (e.g., DMEM) or phosphate-buffered saline (generally referred to as PBS), and then mixed to achieve a ratio of splenocytes to myeloma cells of approximately 5:1 to 10:1 in general, followed by centrifugation. The supernatant is removed, and then the precipitated cell groups are loosened well. Subsequently, 1 ml of a serum-free medium containing 50% (w/v) polyethylene glycol (with a molecular weight of 1000-4000) is added dropwise while agitating the solution. After 10 ml of a serum-free medium has been slowly added, centrifugation is performed. The supernatant is discarded again, the precipitated cells are suspended in an appropriate volume of a normal medium (generally referred to as HAT medium) containing a hypoxanthine-aminopterin-thymidine fluid and human interleukin-6. The suspension is dispensed in each well of a culture plate, and then the cells are cultured in the presence of 5% carbon dioxide gas at 37°C for approximately 2 weeks. The media are appropriately supplemented with HAT media during culture.

When myeloma cells are of a 8-azaguanine-resistant line, that is, a cell line deficient in hypoxanthine-guanine-phosphoribosyltransferase (HGPRT), the myeloma cells that have not fused, and the myeloma cells that have fused to each other are unable to survive in HAT-containing media. On the other hand, fused cells of splenocytes or hybridomas of splenocytes and myeloma cells can survive, but the fused cells of splenocytes have a limited lifetime. Therefore, by continuing culture in HAT-containing media, only hybridomas of splenocytes and myeloma cells can survive.

Hybridomas producing the desired protein encoded by the above introduced nucleic acid sequence can be selected from the obtained hybridomas by ELISA screening using an antibody specific to the desired protein encoded by the above introduced nucleic acid sequence.

### 8. Method for producing protein

The present invention further provides a method for producing a desired protein, comprising causing the production of a desired protein using any of the above chimeric non-human animals or the offspring thereof, the above tissues or cells, or the above hybridomas, and collecting the proteins. Specifically, chimeric non-human animals or offsprings thereof are fed under conditions that enable the expression of an introduced nucleic acid sequence encoding a desired protein. Then the proteins, the expression products, can be collected from the blood, ascites, or the like of the animal. Alternatively, the tissue or the cell derived from a chimeric non-human animal or an offspring thereof, the same that has been immobilized (e.g., hybridomas immobilized by fusion with myeloma cells), or the like is cultured under conditions that enable the expression of an introduced nucleic acid sequence encoding a desired protein. Then the proteins, the expression products, can be collected from the culture product, the culture supernatant, or the like. The expression products can be collected according to a known method such as centrifugation, and then purified by one type of, or a combination of multiple known methods such as ammonium sulfate fractionation, partition chromatography, gel filtration chromatography, adsorbent chromatography (e.g., ion exchange chromatography, hydrophobic interaction chromatography, and hydrophilic chromatography), fractionated thin-layer chromatography, and HPLC.

### 9. Method for analyzing in vivo functions

The present invention further provides a method for analyzing the in vivo functions of a desired protein or a gene encoding the desired protein, comprising comparing the phenotype of the above chimeric non-human animal or an offspring thereof with that of a chimeric non-human animal or the like that has been produced from a corresponding wild-type ES cell containing no nucleic acid sequence encoding the desired protein, and then determining differences among the phenotypes.

According to this method, any trait that appears *in vivo* corresponding to gene transfer is detected by a physicochemical method, whereby *the in vivo* functions of the introduced nucleic acid sequence or the protein can be identified. For example, blood of a chimeric non-human animal produced from an ES cell containing a nucleic acid sequence encoding a desired protein, or an offspring thereof, and blood of a chimeric non-human animal produced from a corresponding wild-type ES cell containing no nucleic acid sequence encoding the above desired protein are collected and then analyzed using a blood cell counter. By comparing the measured concentrations in blood of leucocytes. erythrocytes, platelets, and the like between the above 2 types of chimeric non-human animals, the action of the desired protein encoded by the introduced nucleic acid sequence on the proliferation and differentiation of blood-cell-system cells can be examined. In examples described later, DNA encoding thrombopoietin (TPO) was used as an introduced nucleic acid sequence. In this case, significant increases in platelets (trait) were observed in chimeric mice.

### BRIEF DESCRIPTION OF THE DRAWINGS

Fig. 1 shows the structure of a knock-in basic vector pKIκ. Cκ: mouse Igκ gene, IRES: internal ribosomal entry site, *Sal* I: cloning site, Cκ polyA: mouse Igκ polyA signal, Puro^{r}: puromycin-resistance gene, DT-A: diphtheria toxin A chain gene, and PUC18: cloning vector.
Fig. 2 shows the structure of a plasmid pΔCκNeo. pSTneoB: neomycin-resistance gene, DT-A: diphtheria toxin A chain gene, and pUC18: cloning vector.
Fig. 3 shows the structure of a knock-in vector pKI-I-1. Promoter 1: mouse Igκ promoter region gene 1, MCS: multi-cloning site, Cκ: mouse Igκ gene, SV40 polyA: SV40 PolyA signal, Cκ polyA: mouse Igκ polyA signal, Puro: puromycin-resistance gene, DT-A: diphtheria toxin A chain gene, and pUC18: cloning vector.
Fig. 4 shows the structure of a knock-in vector pKI-I-2. Promoter 2: mouse Igκ promoter region gene 2, MCS: multi-cloning site, C_{κ}: mouse Ig_{κ} gene, SV40 polyA: SV40 PolyA signal, Cκ polyA: mouse Igκ polyA signal, Puro:
puromycin-resistance gene, DT-A: diphtheria toxin A chain gene, and pUC18: cloning vector.
Fig. 5 shows the structure of a knock-in vector pKI-II-1. Promoter 1: mouse Igκ promoter region gene 1, MCS: multi-cloning site, Cκ: mouse Igκ gene, Cκ polyA: mouse Igκ polyA signal, Puro: puromycin-resistance gene, DT-A: diphtheria toxin A chain gene, and pUC18: cloning vector.
Fig. 6 shows the structure of a knock-in vector pKI-II-2. Promoter 2: mouse Igκ promoter region gene 2, MCS: multi-cloning site, Cκ: mouse Igκ gene, Cκ polyA: mouse Igκ polyA signal, Puro: puromycin-resistance gene, DT-A: diphtheria toxin A chain gene, and pUC 18: cloning vector.
Fig. 7 shows the structure of a vector for knocking out mouse FGF23, pBlueLAB-LoxP-Neo-DT-A-3'KO-5'KO. 5'KO: FGF23 knock-out vector 5' homologous region, Neo^{r}: neomycin-resistance gene, EX1: FGF23 exon 1 partial region (+60 to +211), 3'KO: FGF 23 knock-out vector 3' homologous region, DT-A: diphtheria toxin A chain gene, and pBluescript: cloning vector.

### BEST MODE OF CARRYING OUT THE INVENTION

A preferred embodiment of the present invention will be described with the system utilizing an immunoglobulin light chain gene as an example.

Immunoglobulin (Ig) is a protein that is produced in the largest quantity among the proteins secreted in serum. For example in a human, immunoglobulin accounts for 10% to 20% of serum proteins, and the concentration thereof reaches 10 to 100 mg/ml. Ig is produced by B cells, and is mainly produced in large quantities by plasma cells that are the terminal form of the differentiation of B cells. Various factors such as high transcription activity in the Ig gene locus, stability of mRNA, and the functions of plasma cells specialized in secretion and production of proteins contribute to high level expression of Ig. Furthermore, in an adult, B cells are developed in the bone marrow, and shift to lymphoid tissues throughout the entire body such as the spleen, Peyer's patches of the small intestine, and the lymph node as they mature. Accordingly, the product of a transgene that is produced under the regulatory region of an Ig gene of a B cell is released into blood or lymph in a manner similar to that of the case of Ig, and is immediately spread out throughout the entire body. The present invention has the advantage of causing the expression of a nucleic acid sequence (structural gene) encoding a desired protein utilizing the Ig expression system that provides such high-level expression.

A position into which a transgene is inserted for efficient expression thereof is the Ig light chain, and preferably the κ light chain. For example, 95% of mouse immunoglobulins contain the κ light chain, and there is only one constant region gene thereof. On the other hand, the light chain λ is contained in 5% of mouse immunoglobulins, and there are 4 different types of genes, and any one of them may be used. In addition, in heavy chain, there is a total of 7 types of constant regions: µ, y (four types), α, and ε. When it is taken into consideration that a transgene is generally inserted into I position, use of the κ chain is preferred.

As an expression form, it is preferred that a further introduced nucleic acid sequence be expressed under conditions whereby a functional Ig light chain is produced. In an Ig gene locus, it is known that the functional expression of Ig suppresses the expression of the other allele by the mechanism of allelic exclusion. It is thought that mRNA would be made unstable when recombination fails, so that the termination codon appears upstream from the original termination codon. Hence, to maximize the expression of an introduced nucleic acid sequence, a state wherein a functional Ig light chain and the introduced nucleic acid sequence (structural gene) are present in a single mRNA is preferred.

The chimeric non-human animal or an offspring thereof of the present invention preferably contains on the genome a gene wherein an IRES is located downstream of the termination codon of a nucleic acid sequence encoding an immunoglobulin light chain, and a nucleic acid sequence (transgene) encoding a desired protein is located downstream of the IRES. The insertion site of an IRES + transgene is preferably between the termination codon and a polyA addition site of an Ig light chain, and the number of such sites may be multiple. To prevent the secondary structure formed by the IRES sequence from having an adverse effect on the translation of an Ig light chain gene, it is desirable to provide a certain space (e.g., several bp to several 10 bp) between the termination codon of the Ig light chain gene and the IRES.

To alter the genome of an animal so that the genome contains an IRES downstream of an immunoglobulin light chain gene, and it contains a nucleic acid sequence encoding a desired protein downstream of the IRES, a knock-in vector is prepared. Into this vector DNA, the IRES and the nucleic acid sequence encoding a desired protein are inserted. As this vector, pKIκ (see Example 1) is preferred. In the knock-in vector, an appropriate restriction enzyme cleavage site is introduced as an insertion site for insertion of the IRES sequence and the nucleic acid sequence (to be introduced) between the termination codon and the polyA addition site (e.g., in the vicinity of the halfway point) of the light chain, and a DNA (cDNA or genomic DNA) containing a region from the initiation codon to the termination codon of the nucleic acid sequence to be introduced is inserted into the restriction enzyme cleavage site. In addition, preferably, a translational stimulatory sequence, such as a Kozak sequence, can be located upstream of the initiation codon. Furthermore, for the simple identification of homologous recombinants, a drug resistance gene marker, and preferably a puromycin resistance gene, can be previously inserted into the position at which a foreign gene is knocked-in. In this step of preparing a knock-in vector DNA, treatment with polyamines such as spermidine or an analogue thereof is preferably conducted.

Transformation of a non-human animal ES cell using a knock-in vector can be performed by the method described by Shinichi Aizawa (*supra*) or the like. In a manner similar to the method described in the PCT International Application WO 00/10383 pamphlet filed by the applicant (international publication on March 2, 2000), puromycin-resistant clones are picked up, genomic DNA is prepared, and then homologous recombinants are identified by the Southern analysis method. The puromycin-resistance gene in the knock-in vector is derived from a Lox-P Puro plasmid described in the WO 00/10383 pamphlet, and contains in the forward direction a Lox-P sequence at both of its ends. Hence, by the method described in the WO 00/10383 pamphlet, this resistance gene can be removed from the ES cell to which the gene has been knocked-in.

When an immunoglobulin light chain gene is utilized in the present invention, as a deficient host embryo for injection of ES cells, a non-human animal strain which is homologous in disruption of the immunoglobulin heavy chain gene described in the WO 00/10383 pamphlet is preferably used.

The prepared knock-in ES cell is injected into the blastocyst or the 8-cell-stage embryo of the above deficient host embryo using a capillary or the like. This blastocyst or the 8-cell-stage embryo is directly transplanted into the oviduct of a foster parent non-human animal of the same species, or cultured for 1 day for the embryo to develop to a blastocyst, and then, the blastocyst is transplanted into the uterus of a foster parent. Subsequently, the foster parents are fed to give birth, thereby obtaining offsprings.

In a chimeric non-human animal, host-embryo-derived mature B lymphocytes are absent, and only those derived from the knock-in ES cells are present. This is because the immunoglobulin heavy chain knock-out non-human animal that is used as a host embryo is deficient in mature B lymphocytes (B220 positive), so that no immunoglobulins are detected in blood (Tomizuka et al., Proc. Natl. Acad. Sci. U.S.A., 97: 722-727, 2000). Recovery of mature B lymphocytes and the production of antibodies in the chimeric non-human animal due to the contribution of the knock-in ES cells can be detected by the FACS analysis, ELISA method, or the like. Whether or not a nucleic acid sequence inserted into the B cell derived from a knock-in ES cell is expressed depends on whether or not a site-specific recombination reaction takes place in an Ig light chain gene of the allele into which the sequence has been inserted. Specifically, when the Ig light chain gene of the inserted allele is recombined successfully, and the mRNA thereof encodes a functional light chain, the inserted nucleic acid sequence (structural gene) that is present at the same time on the mRNA is also translated into a protein by the action of an IRES. Furthermore, when recombination of the κ chain gene of the inserted allele fails, and a κ chain or a λ chain on the other allele encodes functional light chain, transcription of mRNA encoding an unfunctional κ chain and the introduced nucleic acid sequence takes place, so that the protein derived from the inserted nucleic acid sequence is expressed. The inserted nucleic acid sequence is not expressed when a κ chain or a λ chain of the other allele previously succeeds in functional recombination, and the recombination of an Igκ chain of the inserted allele is shut off by the mechanism of allelic exclusion. In a non-human animal B cells appear in the fetal liver tissue around on day 12 of the prenatal period, and the place for the development of B cells shifts to the bone marrow after birth. In the fetal period, B cells are in an early developmental period; that is, they mainly comprise cells expressing membrane immunoglobulin receptors. Because the number of B cells themselves is lower than in the case of an adult, and the quantity of mRNA encoding an immunoglobulin is low in cells mainly expressing membrane Ig, it is thought that the expression of the inserted nucleic acid sequence is also at a very low level compared with the case of an adult. Antibody production begins to increase at the weanling period (3 weeks old), and this is inferred to be due to increased plasma cells at the terminal differentiation stage of B cells. Subsequently, B cells shift to the lymphoid tissue such as the spleen, the lymph node, and Payer's patches of the small intestine, and then express antibodies and the inserted nucleic acid sequence. The desired protein encoded by the inserted nucleic acid sequence is secreted in blood or lymph to spread throughout the entire body, in a manner similar to that of immunoglobulin.

The expression of an introduced nucleic acid sequence in B cells is confirmed as described below. The expression of polycistronic mRNA containing the transgene is detected by the RT-PCR method, the Northern blot method, or the like using mRNA derived from a tissue or a cell population, such as a spleen containing B cells and peripheral blood nucleated cells. The expression at the protein level can be detected utilizing enzyme immunoassay, the Western blot method, or the like using chimeric mouse serum, when a specific antibody against a desired protein encoded by the introduced nucleic acid sequence is already present. In addition, if the DNA encoding a introduced nucleic acid sequence is altered properly, and a sequence encoding tag peptide detectable with an antibody is added to the DNA, the expression of the introduced nucleic acid sequence can be detected using an antibody or the like against the tag peptide.

The chimeric non-human animal obtained as described above expresses the introduced nucleic acid sequence encoding the desired protein efficiently and reliably at high levels. This high efficiency is as described above, and is mainly based on the following points:
(1) By the use of B-lymphocyte-deficient host embryos, all the B lymphocytes of a chimeric animal are derived from ES cells regardless of chimerism.
(2) Homologous recombination occurs in an Igκ gene locus at an efficiency of 5% or more.
(3) An expression system of immunoglobulins is utilized.
(4) The expression of immunoglobulin is very low in the early developmental stage, and shows an explosive increase on and after weanling stage. Thus, even when a gene whose high level expression causes embryonic lethality is introduced, the functions of the gene in an adult can be examined.

### EXAMPLE

The present invention will be explained specifically in the following examples, but is not limited to these examples.

### [Example 1] Construction of knock-in vector pKIκ

### (1) Preparation of a fragment in the vicinity of a cloning site

A restriction enzyme recognition sequence (*Sal* I recognition sequence) for the insertion of an internal ribosomal entry site (IRES) and a nucleic acid sequence encoding a desired protein (to be introduced) is introduced between the termination codon portion and a polyadenylation signal (PolyA signal) of a mouse immunoglobulin kappa chain (Igκ) gene. Then a puromycin-resistance gene is inserted downstream of the PolyA signal, thereby preparing a genomic fragment. The method is specifically described below.

### (1.1) Preparation of upstream fragment of a cloning site

The following DNAs were synthesized from the nucleotide sequence encoding a mouse IgGκ gene obtained from GenBank (NCBI, U.S.A.).
igkc1 : atctcgaggaaccactttcctgaggacacagtgatagg (SEQ ID NO: 1)
igkc2: atgaattcctaacactcattcctgttgaagctcttgac (SEQ ID NO: 2)

An *Xho* I recognition sequence was added to the terminus of igkc1 that was the 5' primer, and an *Eco*R I recognition sequence was added to igkc2 that was the 3' primer. A reaction solution was prepared using TakaRa LA-Taq (TAKARA BIO INC., Japan) according to the instruction by the manufacturer. To 50 µL of the reaction solution, 10 pmol of each primer, and 25 ng of pBluescript SKII(+) (TOYOBO, Japan) to which a λ-clone-derived DNA fragment containing Ig light chain Cκ-Jκ had been cloned (WO 00/10383 pamphlet) as a template was added. The solution was kept at 94°C for 1 minute, and then subjected to 25 cycles of amplification, each cycle consisting of 94°C for 30 seconds and 68°C for 3 minutes. The obtained reaction solution was subjected to phenol/chloroform extraction, ethanol precipitation, and then digestion with *Eco*R I and *Xho* I. DNA fragments were separated on 0.8% agarose gel electrophoresis, target fragments were collected using GENECLEAN II Kit (Qbiogene, Inc., U.S.A.), and then an amplification fragment A was obtained, After digestion with *Eco*R I and *Xho* I, the amplification fragment A was inserted into a pBluescript2 KS-vector (Stratagene, U.S.A.) that had been dephosphorylated with *Escherichia coli* alkaline phosphatase, and then the vector was transformed into *Escherichia coli* DH5α. DNA was prepared from the obtained transformant, and then the nucleotide sequence was confirmed, thereby obtaining a plasmid plgCκA.

### (1.2) Preparation of downstream fragment of cloning site

The following DNAs were synthesized from the nucleotide sequence encoding a mouse IgGK gene obtained from GenBank (NCBI, U.S.A.).
igkc3 : atgaattcagacaaaggtcctgagacgccacc (SEQ ID NO: 3)
igkc4: atggatcctcgagtcgactggatttcagggcaactaaacatt (SEQ ID NO: 4)

An *Eco*R I recognition sequence was added to the terminus of igkc3, which was the 5' primer, and *Bam*H *I, Xho* I, and *Sal* I recognition sequences were added to the 5' side of igkc4, which was the 3' primer. A reaction solution was prepared using TakaRa LA-Taq (TAKARA BIO INC., Japan) according to the instructions by the manufacturer. To 50 µL of the reaction solution, 10 pmol of each primer, and 25 ng of pBluescript SKII(+) (TOYOBO, Japan) to which a λ-clone-derived DNA fragment containing Ig light chain Cκ-Jκ had been cloned (WO 00/10383 pamphlet) as a template was added. The solution was kept at 94°C for 1 minute, and then subjected to 25 cycles of amplification, each cycle consisting of 94°C for 30 seconds, 55°C for 30 seconds, and 72°C for I minute. The obtained reaction solution was subjected to phenol/chloroform extraction, ethanol precipitation, and then digestion with *Eco*R I and *Bam* HI. DNA fragments were separated on 0.8% agarose gel electrophoresis, target fragments were collected using GENECLEAN II Kit (Qbiogene, Inc., U.S.A.), and then an amplification fragment B was obtained. After digestion with EcoR I and *Bam*H I, the amplification fragment B was inserted into a pIgCκA vector that had been dephosphorylated with *Escherichia coli* alkaline phosphatase, and then the vector was transformed into *Escherichia coli* DH5α. DNA was prepared from the obtained transformant, and then the nucleotide sequence was confirmed, thereby obtaining a plasmid pIgCκAB.

### (1.3) Insertion of puromycin-resistance gene

Lox-P Puro plasmid (WO 00/10383 pamphlet) was digested with *EcoR I* and *Xho* I, and then converted to blunt ends with T4 DNA polymerase. DNA fragments were separated on 0.8% agarose gel electrophoresis, and then DNA fragments containing loxP-puromycin-resistance genes were collected using GENECLEAN II Kit (Qbiogene, Inc., U.S.A.). The obtained loxP-puromycin-resistance gene fragment was inserted into the pIgCκAB plasmid that had been digested with *Sal* I and converted into blunt ends, and then the plasmid was transformed into *Escherichia coli* DH5α. DNA was prepared from the obtained transformant, and then the nucleotide sequence of the ligated portion was confirmed, thereby obtaining a plasmid pIgCκABP.

### (1.4) Insertion of IRES gene

The following DNAs were synthesized from the nucleotide sequence encoding an IRES gene derived from encephalomyocarditis virus obtained from GenBank (NCBI, U.S.A.).
eIRESFW : atgaattcgcccctctccctccccccccccta (SEQ ID NO: 5)
esIRESRV: atgaattcgtcgacttgtggcaagcttatcatcgtgtt (SEQ ID NO: 6)

An *Eco*R I recognition sequence was added to the terminus of eIRESFW, which was the 5' primer, and *EcoR* I and *Sal* I recognition sequences were added to the 5' side of esIRESRV, which was the 3' primer. A reaction solution was prepared using TakaRa LA-Taq (TAKARA BIO INC., Ltd, Japan) according to the instructions by manufacturer. To 50 µL of the reaction solution, 10 pmol of each primer and 150 ng of the pIREShyg plasmid (Clontech, U.S.A.) as a template were added. The solution was kept at 94°C for 1 minute, and then subjected to 25 cycles of amplification, each cycle consisting of 94°C for 30 seconds, 55°C for 30 seconds, and 72°C for 1 minute. The obtained reaction solution was subjected to 0.8% agarose gel electrophoresis so as to separate DNA fragments, and target fragments were collected using GENECLEAN II Kit (Qbiogene, Inc., U.S.A.). The obtained DNA fragment was inserted into the pGEM-T vector (Promega, U.S.A.), and then the vector was transformed into *Escherichia coli* DH5α. DNA was prepared from the obtained transformant, and then the nucleotide sequence was confirmed, thereby obtaining a plasmid IRES-Sal/pGEM. This plasmid was digested with EcoR I, DNA fragments were separated on 0.8% agarose gel electrophoresis, and then target fragments were collected using GENECLEAN II Kit (Qbiogene, Inc., U.S.A.). The obtained IRES gene was inserted into the pIgCκABP plasmid digested with *Eco*R I, and then the plasmid was transformed into *Escherichia coli* DH5α. DNA was prepared from the obtained transformant, and then the nucleotide sequence of the ligation portion was confirmed, thereby obtaining a plasmid pIgCκABPIRES.

### (2) Preparation of pΔCκSal plasmid

After digestion of a targeting vector plasmid for immunoglobulin gene light chain κ knock-out described in the WO 00/10383 pamphlet with *Sac* II, partial digestion treatment with *Eco*R I was performed. The remaining 14.6 Kb of DNA after excision of the LoxP-PGKPuro portion was separated on 0.8% agarose gel electrophoresis, and then collected using GENECLEAN II Kit (Qbiogene, Inc., U.S.A.). By insertion of the following synthetic DNAs into the obtained DNA, a *Sal* I recognition sequence was introduced.
Sal 1plus : agtcgaca (SEQ ID NO: 7)
Sal Iminus: aatttgtcgactgc (SEQ ID NO: 8)

The obtained plasmid was transformed into *Escherichia coli* DH5α, and then DNA was prepared from the obtained transformant, thereby obtaining a plasmid pΔCκSal.

### (3) Construction of knock-in vector pKIκ

The plgCκABPIRES plasmid obtained in (1.4) above was digested with *Xho* I, DNA fragments were separated on 0.8% agarose gel electrophoresis, and then DNA fragments containing Cκ-IRES-loxP-puromycin-resistance genes were collected using GENECLEAN II Kit (Qbiogene, Inc., U.S.A.). The pCκSal prepared in (2) above was digested with *Sal* I, and then dephosphorylated with *Escherichia coli* alkaline phosphatase. Into the resultant pCκSal, the above DNA fragment was inserted, and then the plasmid was transformed into *Escherichia coli* DH5α. DNA was prepared from the obtained transformant, and then the nucleotide sequence of the ligated portion was confirmed, thereby obtaining a plasmid pKIκ. The structure of pKIκ is shown in Fig. 1. The outline of the pKIκ vector structure is as follows.

Specifically, the vector comprises, from the 5' side, mouse Igκ genomic DNA containing the enhancer sequence existing downstream of a J fragment and the Igκ light chain C region (*Eco*R I to *Eco*R I, approximately 5.3 Kb), an internal ribosomal entry site (approximately 0.6 Kb), the cloning site (*Sal* I) located immediately following the 3' end of the internal ribosomal entry site, a mouse Cκ polyA signal region (approximately 0.3 Kb), a puromycin-resistance gene cassette (approximately 1.8 Kb), a mouse Igκ genomic DNA located downstream of a mouse Cκ polyA signal region (approximately 7.7 Kb), a diphtheria toxin A (DT-A) gene cassette (approximately 1.0 Kb), and PUC18 DNA (approximately 2.7 Kb). Knock-in ES cells are prepared by conducting, in a similar manner, the procedures described in Example 2 and the following examples described later by using the above described knock-in vector. In the knock-in ES cell, an internal ribosomal entry site is located immediately following the termination codon of the Cκ structural gene of mouse Igκ, and a desired structural gene can be located at a cloning site located immediately following the 3' end of the internal ribosomal entry site. Furthermore, a mouse Cκ polyA signal region can be located immediately following the desired structural gene. By the use of an IRES, the translational efficiency of a structural gene located downstream of the termination codon of the Cκ structural gene of mouse Igκ is improved drastically (Mizuguchi et al., Molecular Therapy Vol. 1, No. 4, 2000, 376-382.).

### [Example 2] Insertion of TPO gene into pKIκ

### (1) Preparation of mouse thrombopoietin (TPO) gene for insertion into pKIκ

The following DNAs were synthesized from the nucleotide sequence encoding a mouse TPO gene (International Publication WO 95/18858 pamphlet).
tpoN: CCGCTCGAGCGGCCACCATGGAGCTGACTGATTTGCT (SEQ ID NO: 9)
tpoR: CCGCTCGAGCGGCTATGTTTCCTGAGACAAATTCC (SEQ ID NO: 10)

An *Xho* I recognition sequence and a Kozak sequence were added to the 5' side of the terminus of tpoN, which was the 5' primer, and *Xho* I recognition sequence was added to the terminus of tpoR, which was the 3' primer.

A reaction solution was prepared using TaKaRa LA-Taq (TAKARA BIO INC., Japan) according to the instructions by manufacturer. To 100 µL of the reaction solution, 10 pmol of each primer and 100 pg of Marathon-Ready cDNA (Mouse Liver, Clontech, U.S.A.) as a template were added. The solution was kept at 94°C for 30 seconds, and then subjected to 25 cycles of amplification, each cycle consisting of 98°C for 1 second, 50°C for 30 seconds, and 72°C for 1 minute. The obtained reaction solution was subjected to 0.8% agarose gel electrophoresis, so as to separate PCR amplification fragments, and then target DNA fragments were collected using a QIAquick Gel Extraction Kit (QIAGEN, Germany). The obtained DNA fragments were treated with *Xho* I at 37°C for 3 hours, and then collected using the QIAquick PCR Purification Kit (QIAGEN, Germany). pBluescript II SK(-) (TOYOBO, Japan) was treated with *Xho* I and then with CIAP (TAKARA BIO INC., Japan), thereby obtaining a pBlueXhoICIAP plasmid. 42 ng of the pBlueXhoIClAP plasmid, 108 ng of the PCR amplification fragment obtained by treatment with *Xho* I, and 10 µL of a DNA Ligation Kit Ver. 2, Solution I (TAKARA BIO INC., Japan) were mixed. After incubation at 16°C for 30 minutes, the product was transformed into *Escherichia coli* DH5α. A plasmid DNA (pBlueTPO) was prepared from the obtained transformant, and then the nucleotide sequence was confirmed. 9.6 µg of pBlueTPO was digested with *Xho* I at 37°C for 4 hours, and then TPO fragments were collected by 0.8% agarose gel electrophoresis using a QIAquick Gel Extraction Kit (QIAGEN, Germany). The collected TPO fragments were separated on 0.8% agarose gel and collected again, thereby preparing a mouse TPO gene for insertion into pKIκ.

### (2) Preparation of mouse TPO gene-inserted knock-in vector

21 µg of pKIκ prepared in Example 1 was digested with *Sal* I at 37°C for 4 hours, and then vectors were collected by ethanol precipitation. The collected vector was dissolved in 10 µL of TE, and then pKIκ-SalI-CIAP dephosphorylated with CIAP (TAKARA BIO INC., Japan) was prepared. 40 ng of dephosphorylated pKIκ-SalI-CIAP, 4 ng of the mouse TPO gene for insertion into pKIκ prepared in (1) above, and 1.8 µL of a DNA Ligation Kit Ver. 2, Solution I (TAKARA BIO INC., Japan) were mixed, incubated at 16°C for 18 hours, and then transformed into *Escherichia coli* XL10-Gold Ultracompetent Cells (TOYOBO, Japan). DNA was prepared from the obtained transformant, the nucleotide sequence was confirmed, and then a mouse TPO gene-inserted knock-in vector was prepared using a QlAfilter Plasmid Kit (QIAGEN, Germany).

### (3) Preparation of mouse TPO gene-inserted knock-in vector for electroporation

150 µg of the mouse TPO gene-inserted knock-in vector prepared in (2) above was digested with *Xho* I at 37°C for 6.5 hours, and then subjected to phenol/chloroform extraction. 2.5 volumes of 100% ethanol and 0.1 volume of 3M sodium acetate were added to the digest, and then the mixture was stored at -20°C for 16 hours. The vectors were linearized with *Xho* I, collected by centrifugation, and then sterilized by the addition of 70% ethanol. 70% ethanol was removed in a clean bench, and then air-dried for 1 hour. An HBS solution was added to obtain a 0.5 µg/µL DNA solution and then stored at room temperature for 1 hour, so that a mouse TPO gene-inserted knock-in vector for electroporation was prepared.

### [Example 3] Preparation of TPO knock-in ES cell line

To prepare a mouse ES cell line wherein TPO-cDNA was inserted downstream of an immunoglobulin κ light chain gene by homologous recombination, the TPO knock-in vector prepared in Example 2 was linearized with an *Xho* I restriction enzyme (TAKARA BIO INC., Japan), and then transfected into a TT2F mouse ES cell (Yagi et al., Analytical Biochem., 214: 70, 1993) by an established method (Shinichi Aizawa, Bio Manual Series 8, Gene Targeting, YODOSHA, 1995).

The method for culturing TT2F was performed as described (Shinichi Aizawa, *supra*). Feeder cells used herein were G418-resistant primary cultured cells (purchased from Invitrogen, Japan) treated with mitomycin C (SIGMA, U.S.A.). First, the propagated TT2F cells were trypsinized, and then suspended in HBS to achieve a concentration of 3×10⁷ cells/ml. 0.5 ml of the cell suspension was admixed with 10 µg of the vector DNA, and then the mixture was subjected to eleetroporation using a Gene Pulser Cuvette (electrode distance: 0.4 cm; Bio-Rad, U.S.A.) (capacity: 960 µF; voltage: 240 V, room temperature). The cells that had been subjected to electroporation were suspended in 10 ml of an ES medium (Shinichi Aizawa, *supra*), and then inoculated onto a single 100 mm plastic dish for tissue culture (Falcon, Becton Dickinson, U.S.A.) into which feeder cells had been previously inoculated. 36 hours later, the medium was changed with an ES medium containing 0.8 µg/ml puromycin (SIGMA, U.S.A.). A total of 124 colonies were picked up from among colonies that had appeared 7 days later, and each colony was allowed to grow to reach confluence in a 24-well plate. Two-thirds of the colonies were suspended in 0.2 ml of a medium for storage (ES culture media +10% DMSO, SIGMA, U.S.A.), and then stored at -80°C. The remaining one-third of the colonies were inoculated onto a 12-well gelatine-coated plate, and then cultured for 2 days, thereby preparing genomic DNA from 10⁶-10⁷ cells using Puregene DNA Isolation Kits (Gentra Systems, U.S.A.). These G puromycin-resistant TT2F cell genomic DNAs were digested with a restriction enzyme *Eco*R I (TAKARA BIO INC., Japan), and then separated by agarose gel electrophoresis. Subsequently, Southern blot was performed, and then homologous recombinants were detected using as a probe a DNA fragment (*Xho* I to *Eco*R I; approximately 1.4 kb) of the 3' end of the Ig light chain Jκ-Cκ genomic DNA used in the procedures described in the WO 00/10383 pamphlet (see Example 48). As a result, 2 out of 124 clones (2%) were homologous recombinants. In wild-type TT2F cells, a single band was detected by digestion with *Eco*R I. It is predicted that in the homologous recombinants, a new band will appear below the band (WO 00/10383 pamphlet (see Example 58)) in addition to the single band. In the puromycin-resistant clones #6 and #11, this new band was confirmed. Hence, in these clones, TPO-cDNA had been inserted downstream of immunoglobulin κ chain gene on one allele.

### [Example 4] Production of chimeric mouse using TPO knock-in mouse ES cell clone and host embryo derived from B-lympliocyte-deficient mouse strain.

The homozygote of immunoglobulin µ chain gene knock-out is deficient in functional B-lymphocytes, and no antibodies are produced (Kitamura et al., Nature, 350: 423-426, 1991). Embryos obtained by the crossing of female and male mice of the above homozygotes bred in a clean environment were utilized as hosts for the production of chimeric mice in this example. In this case, most functional B-lymphocytes in chimeric mice are derived from ES cells that have been injected. In this example, mice obtained by 3 or more backerossings of the immunoglobulin µ chain gene knack-out mouse described in the report of Tomizuka et al., (Proc. Natl. Acad. Sci. U.S.A., 97: 722-7, 2000) and the mice of MCH (ICR) strain (CLEA JAPAN, INC., Japan) were used for preparing host embryos.

The puromycin-resistant TT2F cell clone #11 that had been obtained in the above Example 3 and confirmed to have TPO-cDNA inserted downstream of an immunoglobulin κ chain gene was prepared from frozen stock. 8 to 10 of these cells were injected into a 8-cell-stage embryo obtained by the crossing of the female and male mice of the above immunoglobulin µ chain knock-out mouse homozygotes. The embryos were cultured overnight in ES media (Shinichi Aizawa, Bio Manual Series 8, Gene Targeting, YODOSHA, 1995) to develop into blastocysts. Approximately 10 injection embryos were transplanted per uterus on the one side of a foster parent MCH (ICR) mouse (CLEA JAPAN, INC., Japan) at 2.5 days after pseudopregnancy treatment. As a result of the transplantation of a total of 180 injection embryos, 16 chimeric mouse offspring were born. Mice were determined to be chimeric if they had coat color wherein wild color (dark brown) derived from TT2F cells was observed with a white color derived from the host embryos. 10 out of 16 offspring born had clear wild color portions in their coat colors, that is, wherein the contribution of ES cells was observed. The highest contribution rate was 100% observed in 2 mice. This result showed that the puromycin-resistant TT2F cell clone #11 having TPO-cDNA inserted downstream of the immunoglobulin κ chain gene possessed chimera formation ability, that is, had the capability of differentiating into a normal tissue of a mouse.

### [Example 5] Increase of platelet counts in TPO knock-in ES cell-derived chimeric mouse

Blood was collected from the retro-orbital sinus of a total of 10 chimeric 6-week-old mice derived from TPO knock-in ES cell clone # 1 1 prepared as in the above Example 4 (chimerism of 100% to 5%) and 5 non-chimeric 6-week-old mice. The number of blood cells of peripheral blood was counted using a blood cell counter (manufactured by SYSMEX CORPORATION, Japan, F-820). In the chimeric mouse group, an average of an 11.04-fold increase in the number of platelet counts was observed compared with the case of non-chimeric mice, regardless of chimerism. In the chimeric mouse group, an average of a 2.03-fold increase in the number of leuckocytes was similarly observed; however, conversely, an average of a 0.76-fold decrease was observed in the number of erythrocyte counts.

Therefore, the protein encoded by TPO gene was thought to have a function to control the number of platelet counts in blood. This result is consistent with the conventionally shown function of TPO gene products (Kato et al., Department of Blood and Tumor (Ketsueki·Shuyo-ka) 33: 1-10, 1996). Hence, it was shown that the method described in the present invention is useful for analyzing *the in vivo* functions of a gene and of gene products.

### [Example 6] Preparation of TPO-producing hybridoma from TPO knock-in ES cell-derived chimeric mouse

On week 10 after birth, the spleens were collected from the chimeric mice TPO (113) (chimerism of 70%), for which increases in platelets had been observed in the above Example 5. Cell fusion of splenocytes of the spleens and myeloma cells was conducted, thereby preparing hybridomas. The method for preparing hybridomas was conducted according to the established method (Ando, Introduction for Monoclonal Antibody Experimental Protocols (Monoclonal Ko-tai Jikken So-sa Nyumon), Kodansha Scientific, 1991), and SP2/0 (RIKEN GENE BANK, RCB0209) was used for myeloma cells. The thus prepared hybridomas were plated onto three 96-well plates. After 10 days of culture, the culture supernatant was analyzed by the ELISA method. The ELISA method was conducted as described in Nishiyama *et al.* (Thromb Haemost 85: 152-1 59, 2001). First, anti-mouse TPO rabbit IgG antibodies were incubated overnight at 4°C to immobilize onto a microtiter plate (Sumilon, Sumitomo Bakelite, Japan), and then the plates were washed. Next, the hybridoma supernatant was added to the plates, and the plates were incubated overnight at 4°C, and then washed. The mouse TPO was finally detected using a biotinylated anti-mouse TPO avian IgG antibody, an alkaline phosphatase-labeled streptavidin, and LumigenPPD (Wako, Japan). As a result, expression of mouse TPO was detected in approximately 15% of the wells wherein HAT-resistant hybridomas were present. Furthermore, when the expression of Igκ chain was examined by the ELISA method, approximately 25% of the HAT-resistant wells was determined as Igκ chain positive, and approximately 60% of Igκ-positive wells was determined as mouse TPO positive. The TPO concentration in the cloned TPO-positive hybridoma supernatant was approximately 10 ng/ml when quantified by the above-described ELISA method.

### [Example 7] Genetic transmission of TPO knock-in immunoglobulin κ chain gene locus from TPO knock-in ES cell-derived chimeric mouse

To determine whether offpsrings derived from ES cells were born by the crossing of male MCH (ICR) strain mouse (CLEA Japan, Inc., Japan) with a female mouse TPO (108) among the TPO knock-in ES cell clone #11-derived chimeric mice produced in the above Example 4 which had shown 100% chimerism, an examination was carried out. By the crossing, wild color offspring mice were born from eggs derived from TT2F cells (wild color, dominance) in the chimeric mice and fertilized with sperms derived from MCH (ICR) male mice (albino, recessive); white offspring mice were born from eggs derived from MCH (ICR) in the chimeric mice and fertilized with sperms derived from MCH (ICR) male mice (albino, recessive). All the 7 offspring mice in total obtained by the crossings showed wild color that was coat color derived from ES cells, indicating the efficient transmission of TPO knock-in ES cells to the germ lines. When the number of platelets was counted in a manner similar to that in the above Example 5 for seven 3-week-old wild colored offspring mice, significant increases (5x10⁶/µl or more) in the number of platelets were observed in 3 out of 7 mice (43%). TPO-cDNA had been inserted into an immunoglobulin gene locus on only one allele in the ES cells. Thus, the probability of carrying the TPO gene inserted in the wild colored mice is inferred to be 50%. The above result is roughly consistent with this inference, showing the genetic transmission of the introduced TPO gene. The above result shows that according to the method described in this specification, a mouse strain expressing a desired secretory protein in a cell or a tissue that is different from the site at which the protein is originally expressed can be established.

### [Example 8] Insertion of human CD20 gene to pKIκ

### (1) Preparation of human CD20 gene for insertion into pKIκ

The following DNAs were synthesized from the nucleotide sequence encoding a human CD20 gene (Genbank, M27394).
20+: CCCTCGAGCCACCATGACAACACCCAGAAATTCAG (SEQ ID NO: 11)
20-: GGGTCGACTTAAGGAGAGCTGTCATTTTC (SEQ ID NO: 12)

The amplification of the human CD20 gene was carried out using Quick Clone cDNA, human spleen (Clontech, U.S.A.) as a template in a manner similar to that in the above Example 2. The PCR reaction solution was subjected to 0.8% agarose gel electrophoresis to separate PCR amplification fragments, and then target DNA fragments were collected using a QIAquick Gel Extraction Kit (QIAGEN, Germany). The obtained DNA fragments were treated with *Xho* I at 37°C for 3 hours, and then collected using a QIAquick PCR Purification Kit (QIAGEN, Germany).

pBluescript II SK(-) (TOYOBO, Japan) was treated with *Xho* I, and then with CIAP (TAKARA BIO INC., Japan). 42 ng of the thus obtained pBlueXhoICIAP plasmid, 108 ng of the PCR amplification fragment obtained by treatment with *Xho* I as described above, and 10 µL of a DNA Ligation Kit Ver. 2, Solution I (TAKARA BIO INC., Japan) were mixed, incubated at 16°C for 30 minutes, and then transformed into *Escherichia coli* DH5α. A plasmid DNA (pBlueCD20) was prepared from the obtained transformant, and then the nucleotide sequence was confirmed. 9.6 µg of pBlueCD20 was digested with *Xho* I at 37°C for 4 hours, and then CD20 fragments were collected by 0.8% agarose gel electrophoresis using a QIAquick Gel Extraction Kit (QIAGEN, Germany). The collected CD20 fragments were separated on 0.8% agarose gel and collected again, thereby preparing a human CD20 gene for insertion into pKIκ.

### (2) Preparation of human CD20 gene-inserted knock-in vector

21 µg of pKIκ prepared in Example 1 was digested with *Sal* I at 37°C for 4 hours, and then the vectors were collected by ethanol precipitation. The collected vector was dissolved in 10 µL of TE, and then pKIκ-SalI-CIAP was prepared by dephosphorylation with CIAP (TAKARA BIO INC., Japan).

40 ng of the above KI-SalI-CIAP, 4 ng of the human CD20 gene for insertion into a knock-in vector prepared in the above (1), and 1.8 µL of a DNA Ligation Kit Ver. 2, Solution I (TAKARA BIO INC., Japan) were mixed,

incubated at 16°C for 18 hours, and then transformed into *Escherichia coli* XL10-Gold Ultracompetent Cells (TOYOBO, Japan). DNA was prepared from the obtained transformant, and then the nucleotide sequence was confirmed. A human CD20 gene-inserted knock-in vector was then prepared using a QlAfilter Plasmid Kit (QIAGEN, Germany).

### (3) Preparation of human CD20 gene-inserted knock-in vector for Electroporation

150 µg of the human CD20 gene-inserted knock-in vector prepared in (2) was digested with *Xho* I (37°C, 6.5 hours) using a spermidine-added (1 mM, pH 7.0, SIGMA, U.S.A.) buffer (Roche Diagnostics, Japan, H buffer for restriction enzyme), or a spermidine-free buffer (Roche Diagnostics, Japan, H buffer for restriction enzyme). After phenol/chloroform extraction, 2.5 volumes of 100% ethanol and 0.1 volume of 3M sodium acetate were added, and then the solution was stored at -20°C for 16 hours. The vector linearized with *Xho* I was collected by centrifugation, and then sterilized by the addition of 70% ethanol. 70% ethanol was removed in a clean bench, and then air-dried for 1 hour. HBS solution was added to result in a 0.5 µg/µL DNA solution, and the resultant was stored at room temperature for 1 hour, thereby preparing a human CD20 gene-inserted knock-an vector for electroporatian.

### [Example 9] Preparation of human CD20 knock-in ES cell clone

To obtain a mouse ES cell clone wherein human CD20-cDNA has been inserted by homologous recombination downstream of an immunoglobulin κ light chain gene, the 2 types of the human CD20 knock-in vectors (spermidine-added and spermidine-free) prepared in the above Example 8 were transfected into each mouse ES cell TT2F (Yagi et al., Analytical Biochem., 214: 70, 1993) according to the established method (Shinichi Aizawa, Bio Manual Series 8, Gene Targeting, YODOSHA, 1995). The method for culturing TT2F cells was performed according to the above-described method (Shinichi Aizawa, *supra*). Feeder cells used herein were G418-resistant primary cultured cells (purchased from Invitrogen, Japan) treated with mitomycin C (SIGMA, U.S.A.). First, the propagated TT2F cells were trypsinized, and then suspended in HBS to be 3×10⁷ cells/ml. 0.5 ml of the cell suspension was mixed with 10 µg of the vector DNA, and then the mixture was subjected to electroporation using a Gene Pulser Cuvette (electrode distance: 0.4 cm, Bio-Rad, U.S.A.) (capacity: 960 µF; voltage: 240V; room temperature). The cells subjected to electroporation were suspended in 10 ml of an ES medium, and then inoculated onto a 100 mm plastic dish for tissue culture (Falcon, Becton Dickinson, U.S.A.), onto which feeder cells had been previously inoculated. 36 hours later, the medium was changed with an ES medium containing 0.8 µg/ml puromycin (SIGMA, U.S.A.). Among the colonies that had appeared 7 days later, 80 (spermidine-free) and 56 (spermidine-added) colonies were picked up in total. Each type of these colonies was grown to reach confluence in a 24-well plate. Two-thirds of these colonies were suspended in 0.2 ml of a medium for storage (ES medium + 10% DMSO, SIGMA, U.S.A.), and then stored at -80°C. The remaining one-third of the colonies were inoculated onto a 1.2-well gelatine-coated plate, and then cultured for 2 days. Genomic DNA was prepared from 10⁶ to 10⁷ cells using Puregene DNA Isolation Kits (Gentra Systems, U.S.A.). These genomic DNAs of G puromycin-resistant TT2F cells were digested with restriction enzyme *Eco*R I (TAKARA BIO INC., Japan), and then separated on agarose gel electrophoresis. Subsequently, Southern blot was performed, and then homologous recombinants were detected using the probe shown in the above Example 3. As a result, 0 out of 80 clones (0%) were homologous recombinants in the spermidine-free case, and 5 out of 56 lines (9%) were homologous recombinants in the spermidine-added case.

### [Example 10] Insertion of human FGF23 gene into knock-in vector

The following DNAs were synthesized from the nucleotide sequence encoding a human FGF23 (fibroblast growth factor 23) gene.
OST311XM: ATCTCGAGCCACCATGTTGGGGGCCCGCCTCAGG (SEQ ID NO: 13)
OST311HX: ATCTCGAGCTAATGATGATGATGATGATGGATGAACTTG GCGAAGGG (SEQ ID NO: 14)

Human FGF23 cDNA was amplified in a manner similar to that in the above Example 2 using as a template a pcDNA vector containing the full-length FGF23 cDNA described in Shimada et al. (Proc. Natl. Acad. Sci. USA, 98: 6500-6505, 2000). The PCR reaction solution was subjected to 0.8% agarose gel electrophoresis to separate PCR amplification fragments, and then target DNA fragments were collected using a QIAquick Gel Extraction Kit (QIAGEN, Germany). The obtained DNA fragments were treated with *Xho* I at 37°C for 3 hours, and then collected using a QIAquick PCR Purification Kit (QIAGEN, Germany). pBluescript II SK(-) (TOYOBO, Japan) was treated with *Xho* I, and then with CIAP (TAKARA BIO INC., Japan) to obtain a pBlueXhoICIAP plasmid. 42 ng of the pBlueXhoICIAP plasmid, 108 ng of FGF23 cDNA PCR amplification fragment obtained by treatment with *Xho* I as described above, and 10 µL of DNA Ligation Kit Ver. 2, Solution I (TAKARA BIO INC., Japan) were mixed, incubated at 16°C for 30 minutes, and then transformed into *Escherichia coli* DH5α. Plasmid DNA (pBlueFGF23) was prepared from the obtained transformant, and then the nucleotide sequence was confirmed. 9.6 µg of pBlueFGF23 was digested with *Xho* I at 37°C for 4 hours. FGF23 fragments were collected by 0.8% agarose gel electrophoresis using a QIAquick Gel Extraction Kit (QIAGEN, Germany). The collected FGF23 fragments were separated on 0.8% agarose gel and collected again, thereby preparing a human FGF23 gene for insertion into pKIκ.

40 ng of dephosphorylated vector, pKIκ-SalI-CIAP (Example 2), 4 ng of the above human FGF23 gene for insertion into the knock-in vector, and 1.8 µL of DNA Ligation Kit Ver. 2, Solution I (TAKARA BIO INC., Japan) were mixed, incubated at 16°C for 18 hours, and then transformed into *Escherichia coli* XL10-Gold Ultracompetent Cells (TOYOBO, Japan). DNA was prepared from the obtained transformant, and then the nucleotide sequence was confirmed. A human FGF23 gene-inserted knock-in vector was prepared using a QIAfilter Plasmid Kit (QIAGEN, Germany). 150 µg of the human FGF23 gene-inserted knock-in vector was digested with *Xho* I (37°C, 6.5 hours) using each of a spermidine-added (1 mM, pH 7.0, SIGMA, U.S.A.) buffer (Roche Diagnostics, Japan, H buffer for restriction enzyme), and a spermidine-free buffer (described above). After phenol/chloroform extraction, 2.5 volumes of 100% ethanol and 0.1 volume of 3M sodium acetate were added, and then the solution was stored at -20°C for 16 hours. The vectors linearized with *Xho* I were collected by centrifugation, and then sterilized by the addition of 70% ethanol. 70% ethanol was removed in a clean bench, and then the product was air-dried for 1 hour. HBS solution was added to result in a 0.5 µg/µL DNA solution, and the resultant was stored at room temperature for 1 hour, thereby preparing a human FGF23 gene-inserted knock-in vector for electroporation.

### [Example 11] Preparation of FGF23 knock-in ES cell clone

To obtain a mouse ES cell clone wherein human FGF23-cDNA was inserted by homologous recombination downstream of an immunoglobulin κ light chain gene, 2 types (spermidine-added and spermidine-free) of the human FGF23 knock-in vectors for electroporation prepared in the above Example 10 were transfected into a mouse ES cell TT2F (Yagi et al., Analytical Biocbem., 214:70, 1993) according to an established method (Shinichi Aizawa, Bio Manual Series 8, Gene Targeting, YODOSHA, 1995). The method for culturing TT2F was performed according to the above-described method (Shinichi Aizawa, *supra*). Feeder cells used herein were G418-resistant primary cultured cells (purchased from Invitrogen, Japan) treated with mitomycin C (SIGMA, U.S.A.). First, the propagated TT2F cells were trypsinized, and then suspended in HBS to achieve 3×10⁷ cells/ml. 0.5 ml of the cell suspension was mixed with 10 µg of the vector DNA, and then the mixture was subjected to electroporation using a Gene Pulser Cuvette (electrode distance: 0.4 cm, Bio-Rad, U.S.A.) (capacity: 960 µF, voltage: 240 V, room temperature). The cells that had been subjected to electroporation were suspended in 10 ml of an ES medium, and then plated on a single 100 mm plastic dish for tissue culture (Falcon, Becton Dickinson, U.S.A.) onto which feeder cells had been previously inoculated. 36 hours later, the medium was changed with an ES medium containing 0.8 µg/ml puromycin (SIGMA, U.S.A.). Among colonies that had appeared 7 days later, 80 colonies (spermidine-free) and 56 (spermidine-added) colonies were picked up in total, and each colony was allowed to grow to reach confluence in a 24-well plate. Two-thirds of the colonies were suspended in 0.2 ml of a medium for storage (ES media +10% DMSO, SIGMA, U.S.A.), and then stored at -80°C. The remaining one-third of the colonies were inoculated onto a 12-well gelatine-coated plate, and then cultured for 2 days. Then, genomic DNA was prepared from 10⁶-10⁷ cells using Puregene DNA Isolation Kits (Gentra Systems, U.S.A.). These puromycin-resistant TT2F cell genomic DNAs were digested with a restriction enzyme *Eco*R I (TAKARA BIO INC., Japan), and then separated by agarose gel electrophoresis. Subsequently, Southern blot was performed, and then homologous recombinants were detected using the probe shown in the above Example 3. As a result, 0 out of 80 clones (0%) were homologous recombinants in the spermidine-free case, and 3 out of 40 lines (8%) were homologous recombinants in the spermidine-added case. The above results show that the addition of spermidine upon preparation of the vector DNA has an effect of increasing the ratio of homologous recombinants to clones with random insertion.

### [Example 12] Preparation of Cκ chain knock-out vector pΔCκNeo

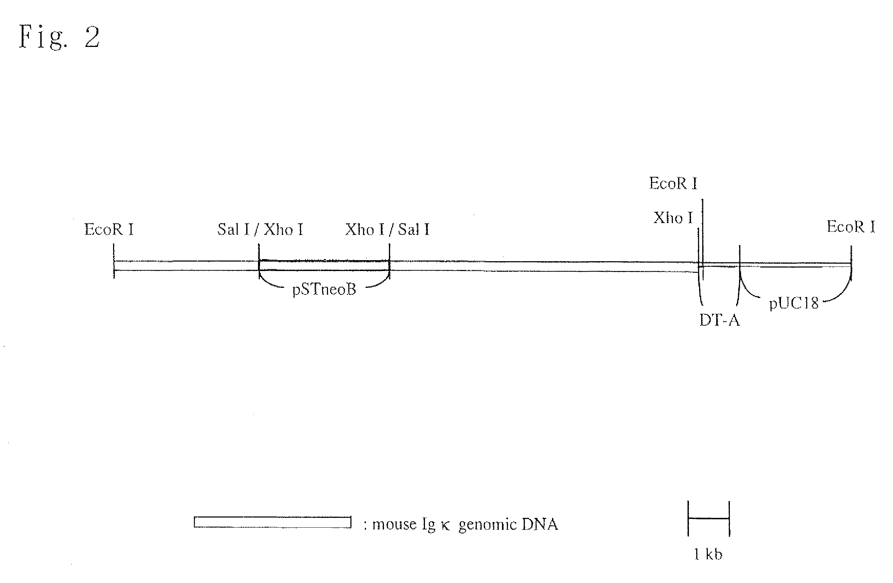

pSTneoB (Katoh et al., Cell Struct. Funct., 12: 575, 1987, Japanese Collection of Research Biologicals (JCRB); Deposit Number: VE039) was digested with restriction enzymes *Xho* I and *Sca* I, and then fragments containing neo-resistance gene was separated on 0.8% agarose gel electrophoresis. Target fragments were collected using GENECEEAN II Kit (Qbiogene, Inc., U.S.A.), and then designated as neo fragment. The above neo fragment was inserted into the pΔCκSal prepared in the above Example 1(2) that had been digested with. *Sal* I, and then dephosphorylated with *Escherichia coli* alkaline phosphatase. The pΔCκSal was then transformed into *Escherichia coli* DH5α, DNA was prepared from the obtained transformant, and then the nucleotide sequence of the ligation portion was confirmed, thereby obtaining a plasmid pΔCκNeo. The structure of pΔCκNeo is shown in Fig. 2.

### [Example 13] Preparation of ESΔΔκneo cell clone

In a manner similar to the methods described in the above Examples 2 and 3, a mouse ES (TT2F) cell was obtained wherein an allele on the one side of an endogenous Igκ gene was disrupted by the pΔCκNeo vector (Example 12). Since the Igκ genomic homologous region within the pΔCκNeo vector was derived from C57BL/6 strain, this vector is inserted preferentially into allele from C57BL/6 in the TT2F cell (derived from F1 of C57BL/6 and CBA strains). Using this ES cell clone (ESΔκneo) and the antibody light chain targeting vector in the WO 00/10383 pamphlet (see Example 76), and the method described in the same, a mouse ES cell (ESΔΔκneo/puro) was obtained wherein a further endogenous Igκ gene allele (CBA) was disrupted. For the ESΔΔκneo/puro clone, the puro gene was removed by Cre recombinase according to the method described in the WO 00/10383 pamphlet (see Example 78). Finally, a G418-resistance and puromycin-sensitive ES cell clone (ESΔΔκneo) was obtained wherein endogenous Igκ genes were disrupted on both alleles. An ES cell clone was obtained using ESΔΔκneo instead of the TT2F cell clone in the establishment of the knock-in ES cell clone described in the above Example 3. Since the homologous region in the Igκ genome within the knock-in vector was derived from the C57BL/6 strain, in the obtained homologous recombinant, this vector is substituted preferentially with a G418-resistance gene in the C57BL/6 allele wherein the G418-resistance gene is present. Hence, the homologous recombinants can be conveniently screened for based on sensitivity to G418. In addition, CBA allele of the endogenous Igκ gene had been already disrupted in the obtained homologous recombinant, so that the Igκ gene of the C57BL/6 allele into which a foreign gene had been inserted by the knock-in vector was expressed exclusively.

### [Example 14] Preparation of type I knock-in vectors pKI-I-1 and pKI-I-2

### (1) Preparation of fragments in the vicinity of cloning site

SV40 PolyA signal, Igκ promoter region 1 or 2, and the multi-cloning site (MCS) of a restriction enzyme recognition sequence for the insertion of a target gene were inserted between the termination codon portion and a polyadenylation signal (PolyA signal) of a mouse immunoglobulin κ chain (Igκ) gene and a puromycin-resistance gene was inserted downstream of the polyA signal to prepare a DNA fragment. The method is specifically shown below.

### (1.1) Preparation of fragment 1 located upstream of cloning site

The following DNAs were synthesized from the nucleotide sequence encoding an SV40 polyA signal region obtained from GenBank (NCBI, U.S.A.).
SVpoly5: GGAATTCAGACATGATAAGATACATTGATGAGTTTGGACAAA (SEQ ID NO: 15)
SVpoly3: CCCAAGCTTTAATCAGCCATACCACATTTGTAGAGGTTTTACTT (SEQ ID NO: 16)

An *EcoR* I recognition sequence was added to the terminus of SVpoly5 that was the 5' primer, and a *Hin*d III recognition sequence was added to the terminus of SVpoly3 that was the 3' primer. A reaction solution was prepared using Takara LA-Taq (TAKARA BIO INC., Japan) according to the instructions by the manufacturer. 10 pmol of each primer and 10 ng of pSTneoB as a template were added to 50 µl of the reaction solution. After being kept at 94°C for 1 minute, the solution was subjected to 25 cycles of amplification, each cycle consisting of 94°C for 30 seconds and 68°C for 30 seconds. The obtained reaction solution was subjected to phenol/chloroform extraction, ethanol precipitation, and then digestion with *Eco*R I and *Hind* III. DNA fragments were separated on 0.8% agarose gel electrophoresis, target fragments were collected as an amplification fragments 1 using GENECLEAN II Kit (Qbiogene, Inc., U.S.A.).

### (1.2) Preparation of fragments 2 and 3 located upstream of cloning site

The following DNAs were synthesized from the nucleotide sequence encoding a mouse Igκ promoter region genes obtained from GenBank (NCBI, U.S.A.).
Cκprom1-5: CCCAAGCTTGAATTAAACAGTTTCAGGGCACATGAAATACTG AG (SEQ ID NO: 17)
Cκprom 1-3: GCTCTAGATTTGTCTTTGAATTTTGGTCCCTAGCTAATTACTG (SEQ ID NO: 18)
Cκprom2-5: CCCAAGCTTTGGTGATTATTCAGAGTAGTTTTAGATGAGTGCAT (SEQ ID NO: 19)
Cκprom2-3: GCTCTAGATTTGTCTTTGAACTTTGGTCCCTAGCTAATTACTA (SEQ ID NO: 20)

A *Hind* III recognition sequence was added to Cκprom1-5 and Cκprom2-5, which were the 5' primers, and an *Xba* I recognition sequence was added to Cκprom1-3 and Cκprom2-3, which were the 3' primers. DNA fragments 2 and 3 were obtained by using a mouse genomic DNA as a template with a primer set (Cκprom1-5 and Cκprom1-3) and a primer set (Cκprom2-5 and Cκprom2-3) using Takara LA-Taq (TAKARA BIO INC., Japan).

### (1.3) Preparation of multi-cloning site fragment 4

The following DNA fragments were synthesized to prepare a multi-cloning site fragment containing *Xba* I, *Sal* I, *Not* I, *Fse* I, *Asc* I, and *Spe* I.

MCS (Xba I/Spe I): GCTCTAGAGTCGACGCGGCCGCGGCCGGCCGGCGCGC
CACTAGTC (SEQ ID NO: 21)

MCS (Spe I/Xba I): GACTAGTGGCGCGCCGGCCGGCCGCGGCCGCGTCGACT
CTAGAGC (SEQ ID NO: 22)

The above 2 DNA fragments were annealed to obtain a DNA fragment 4.

### (1.4) Preparation of fragment 5 located downstream of cloning site

The following DNAs were synthesized from the nucleotide sequence encoding a mouse Igκ gene obtained from GenBank (NCBI, U.S.A.).
CκpolyP5: GACTAGTAGACAAAGGTCCTGAGACGCCACCACCAGCTCCCC (SEQ ID NO: 23)
CκpolyP3: GAAGATCTCAAGTGCAAAGACTCACTTTATTGAATATTTTCTG (SEQ ID NO: 24)

A *Spe* I recognition sequence was added to the terminus of CκpolyP5, which was the 5' primer, and a *Bgl* II recognition sequence was added to the terminus of CκpolyP3 which was the 3' primer. A DNA fragment 5 was obtained by using a mouse genomic DNA as a template with the above primers CκpolyP5 and CκpolyP3 using Takara LA-Taq (TAKARA BIO INC., Japan).

### (1.5) Preparation of DNA fragment I or II containing DNA fragments 1, 2, 4, and 5, or 1, 3, 4, and 5

Each of the above DNA fragments was treated with restriction enzymes to cleave the recognition sequences added to the 5' and 3' sides, and then subjected to phenol/chloroform extraction, and then ethanol-precipitation. The resulting samples were dissolved in TE. DNA fragments 1, 2, 4, and 5, or 1, 3, 4, and 5 treated with restriction enzymes were respectively bound by a ligation reaction. The reaction products were collected by ethanol precipitation, thereby preparing a DNA fragment I or II.

### (2) Construction of type I knock-in vectors, pKI-I-1 and pKI-I-2

### (2.1) Preparation of plgCκΔIRES plasmid

The plgCκABP IRES described in the above Example 1 was treated with restriction enzymes *Eco*R I and *Bgl* II. DNA fragments wherein IoxP-puromycin-resistance gene fragments had not been cleaved with *Bgl* II were separated and collected by agarose gel electrophoresis, thereby obtaining plasmid pIgCκΔIRES fragment.

### (2.2) Preparation of plgCκΔIRES ProI or pIgCκΔIRES ProII

DNA fragment I was inserted into pIgCκΔIRES to prepare pIgCκΔ IRES ProI, and DNA fragment II was inserted into pIgCκΔIRES to prepare pIgCκΔIRES ProII.

### (2.3) Preparation of type I knock-in vectors, pKI-I-1 and pKI-I-2

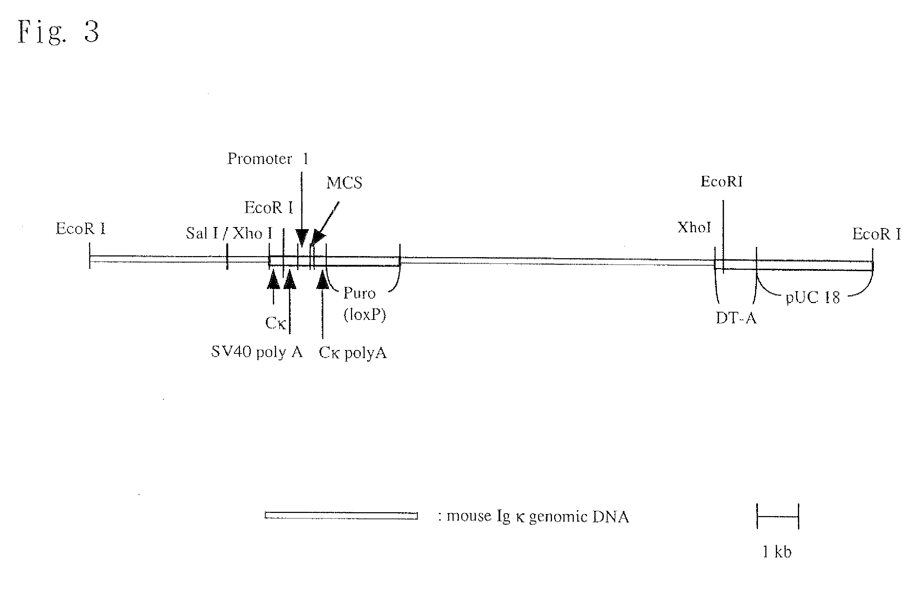

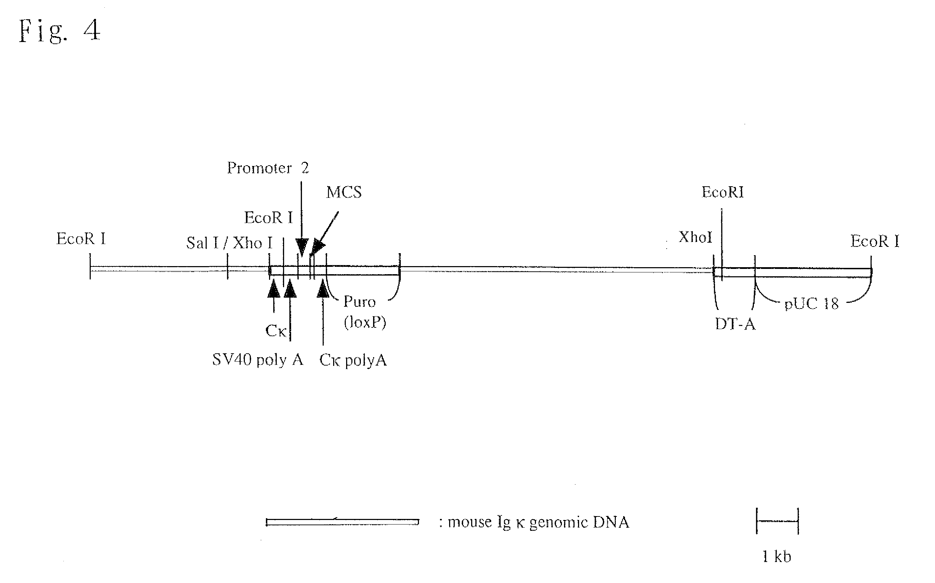

pIgCκΔIRES ProI and pIgCκΔIRES ProII were treated with a restriction enzyme *Xho* I. DNA fragments containing the multi-cloning sites were separated and collected by agarose gel electrophoresis. The thus obtained DNA fragments were inserted into pΔCκSal (Example 1) that had been digested with *Sal* I, and dephosphorylated with *Escherichia coli* alkaline phosphatase, and then the plasmid was transformed into *Escherichia coli* DH5α. DNA was prepared from the obtained transformant, the nucleotide sequence of the ligation portion was confirmed, thereby obtaining plasmids pKI-I-1 and pKI-I-2. The structure of pKI-I-1 and that of pKI-I-2 are shown in Figs. 3 and 4. The outline of the vector structure is as follows.

Specifically, the vector comprises, from the 5' side, mouse Igκ genomic DNA containing the enhancer sequence downstream of a J fragment and Igκ light chain C region *(EcoR* I to *Eco*R 1, approximately 5.3 Kb), SV40 polyA signal region (approximately 0.4 Kb), a region containing a mouse Igκ promoter 1 (approximately 0.2 Kb; in the case of pKI-I-1, and in the case of pKI-I-2, mouse Igκ promoter 2), a region containing a multiple cloning site (MCS; approximately 0.02 Kb), a mouse Cκ polyA signal region (approximately 0.4 Kb), a puromycin-resistance gene cassette (approximately 1.8 Kb), a mouse Igκ genomic DNA (approximately 7.7 Kb) located downstream of a mouse Cκ polyA signal region, a diphtheria toxin A (DT-A) gene cassette (approximately 1.0 Kb), and pUC18 DNA (approximately 2.7 Kb). A knock-in ES cell is prepared by similarly conducting the procedures in the above Example 2 and the following examples by using the above knock-in vector. In a knock-in ES cell, the SV40 polyA signal region was located immediately following the termination codon of the Cκ structural gene of the mouse Igκ, the mouse Igκ promoter 1 (or 2) and a desired structural gene were located immediately following the SV40 polyA signal region, and a mouse Cκ polyA signal region was located immediately following the desired structural gene. By locating the structural gene immediately following the mouse Igκ promoter, it is predicted that the desired gene will be expressed at high levels equivalent to or greater than that in the case of the pKIκ vector using an IRES (Mizuguchi et al., Molecular Therapy Vol.1, No.4, 2000, 376-382.). Knock-in ES cells were prepared by conducting similarly the procedures in the above Example 2 and the following examples using the knock-in vector obtained as described above instead of using pKIκ in the above Example 1.

### [Example 15] Construction of type II knock-in vectors, pKI-II-1 and pKI-II-2

### (1) Preparation of fragment in the vicinity of cloning site

A restriction enzyme recognition sequence multi-cloning site (MCS) for the insertion of a Cκ PolyA signal, a Cκ promoter region 1 or 2, and a target gene was introduced between the termination codon portion and the Cκ polyadenylation signal (PolyA signal) of a mouse immunoglobulin κ chain (Igκ) gene, and a puromycin-resistance gene was introduced downstream of Cκ polyA signal, thereby preparing a genomic fragment. The preparation method is specifically described as follows.

### (1.1) Preparation of fragment 6 located upstream of cloning site

The following DNAs were synthesized from the nucleotide sequence of Cκ polyA signal region obtained from GenBank (NCBI, U.S.A.).

CκpolyT5:    GGAATTCAGACAAAGGTCCTGAGACGCCACCACCAG
CTCCCC (SEQ ID NO: 25)

CκpolyT3:    CCCAAGCTTGCCTCCTCAAACCTACCATGGCCCAGAGA
AATAAG (SEQ ID NO: 26)

An *EcoR* I recognition sequence was added to the terminus of CκpolyT5 which was the 5' primer, and an *Hind* III recognition sequence was added to the terminus of CκpolyT3, which was the 3' primer. A reaction solution was prepared using Takara LA-Taq (TAKARA BIO INC., Japan) according to the instructions by the manufacturer. To 50 µL of the reaction solution, 10 pmol of each primer and 10 ng of mouse genomic DNA as a template were added. The solution was kept at 94°C for 1 minute, and then subjected to 25 cycles of amplification, each cycle consisting of 94°C for 30 seconds and 68°C for 30 seconds. The obtained reaction solution was subjected to phcnol/chlaroform extraction, ethanol precipitation, and then digestion with EcoR I and *Hind* III. DNA fragments were separated on 0.8% agarose gel electrophoresis, target fragments were collected using GENECLEAN II Kit (Qbiogene, Inc., U.S.A.) to obtain an amplification fragment 6.

### (1.2) Preparation of DNA fragment III or IV containing DNA fragments 6, 2, 4, and 5 or 6, 3, 4, and 5

Each of the above DNA fragments was treated with restriction enzymes to cleave the recognition sequences added to the 5' and 3' sides, respectively, and then subjected to phenol/chloroform extraction, and then ethanol-precipitation. The resulting samples were dissolved in TE. DNA fragments 6, 2, 4, and 5, or 6, 3, 4, and 5 treated with restriction enzymes, were bound by ligation reactions, respectively. The reaction products were collected by ethanol precipitation, thereby preparing a DNA fragment III or IV.

### (2) Construction of type II knock-in vectors, pKI-II-1 and pKI-II-2

### (2.1) Preparation of pIgCκΔ IRES ProIII or plgCκΔIRES ProIV

DNA fragment III was inserted into pIgCκΔIRES, so as to prepare pIgCκΔIRES ProIII, and DNA fragment IV was inserted into pIgCκΔIRES, so as to prepare pIgCκΔIRES ProIV.

### (2.2) Preparation of type II knock-in vectors, pKI-II-1 and pKII-I-2

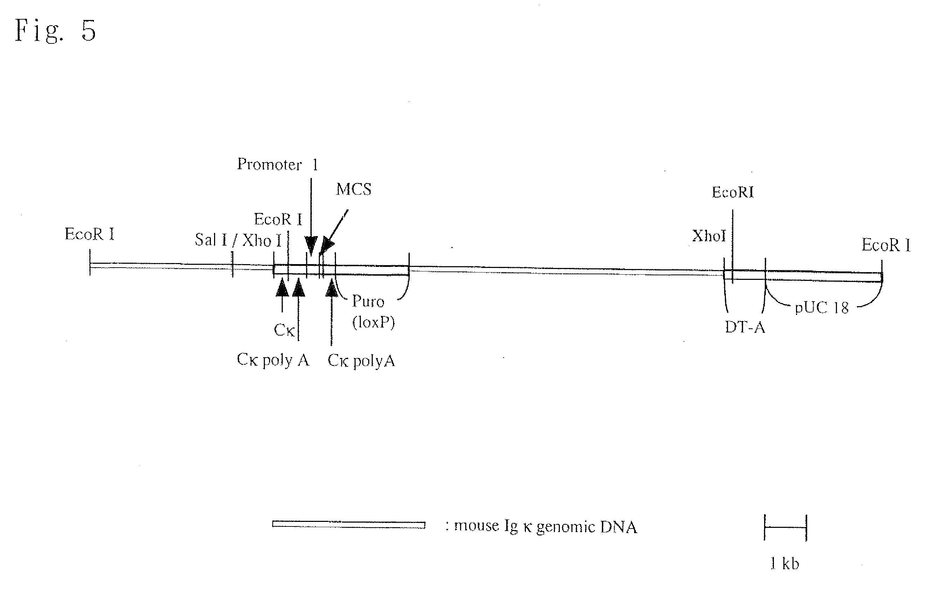

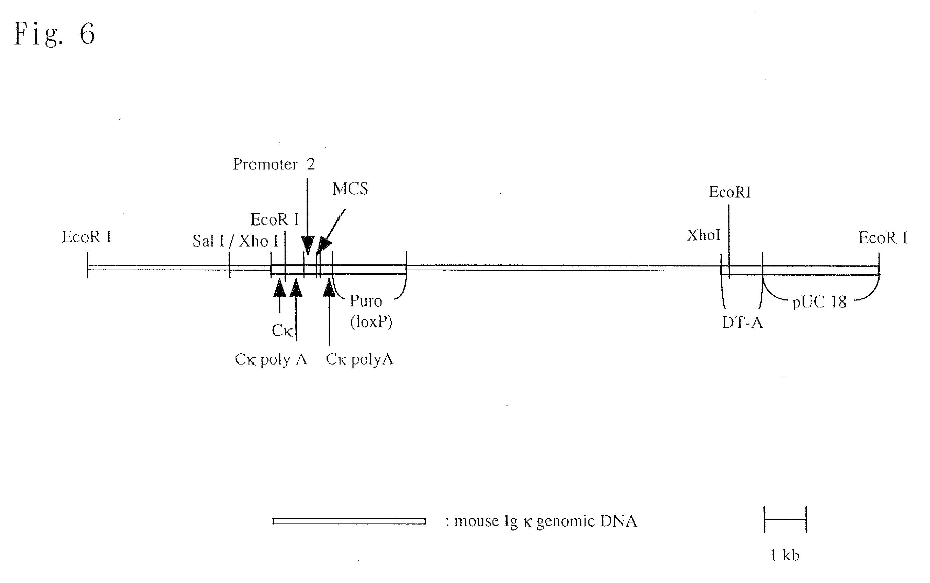

pIgCκΔIRES ProIII and pIgCκΔIRES ProIV were treated with a restriction enzyme *Xho* I. DNA fragments containing the multi-cloning site were separated and collected by agarose gel electrophoresis. The thus obtained DNA fragment was inserted into pCκSal that had been digested with *Sal* I and dephosphorylated with *Escherichia coli* alkaline phosphatase. Then the plasmid was transformed into *Escherichia coli* DH5α. DNA was prepared from the obtained transformant and the nucleotide sequence of the ligation portion was confirmed, thereby obtaining plasmids pKI-II-1 and pKI-II-2. The structure of pKI-II-1 and that of pKI-II-2 are shown in Figs. 5 and 6. The outline of the vector structure is as follows.

Specifically, the vector comprises, from the 5' side, mouse Igκ genomic DNA containing the enhancer sequence downstream of a J fragment and the Igκ light chain C region (*Eco*R I to *Eco*R I, approximately 5.3 Kb), a region containing mouse Cκ polyA (approximately 0.4 Kb), a region containing a mouse Igκ promoter 1 (approximately 0.2 Kb; in the case of pKI-II-1, and in the case of pKI-II-2, mouse Igκ promoter 2), a region containing the multiple cloning site (MCS; approximately 0.02 Kb), a region containing mouse Cκ polyA (approximately 0.4 Kb), a puromycin-resistance gene cassette (approximately 1.8 Kb), a mouse Igκ genomic DNA located downstream of the mouse Cκ polyA (approximately 7.7 Kb), a diphtheria toxin A (DT-A) gene cassette (approximately 1.0 Kb), and pUC18 DNA (approximately 2.7 Kb). A knock-in ES cell is prepared by similarly conducting the procedures in the above Example 2 and the following examples by using the above knock-in vector. In the knock-in ES cell, the mouse Cκ polyA signal region was located immediately following the termination codon of the Cκ structural gene of the mouse Igκ, the mouse Igκ promoter 1 (or 2) and a desired structural gene were located immediately following the mouse Cκ polyA signal region, and a mouse Cκ polyA signal region was located immediately following the desired structural gene. By locating the structural gene immediately following the mouse Igκ promoter, it is predicted that the desired gene will be expressed at high levels equivalent to, or greater than that in the case of the vector pKIκ using an IRES (Mizuguchi et al., Molecular Therapy Vol.1, No.4, 2000, 376-382.). Knock-in ES cells were prepared by conducting similarly the procedures in the above Example 2 and the following examples using the knock-in vector obtained as described above instead of using pKIκ in the above Example 1.

### [Example 16] High level expression of foreign gene in muscle tissue utilizing a myoglobin gene regulatory sequence

Myoglobin is an oxygen-binding heme protein that is present in large quantities in muscle, and the expression thereof is limited to the muscle tissue such as skeletal muscle or the heart (Tomizuka et al., Nature Genet. 16: 133-43, 1997). As described above, by the injection of a mouse ES cell, which contains a foreign gene located so as to be expressed under the regulation of the regulatory sequence of a myoglobin gene, into an embryo derived from a mouse strain deficient in the ability to form muscle tissue (e.g., a myogenin gene-deficient homozygote, Nabeshima et al., Nature 364: 532-5, 1993), the foreign gene can be expressed at high levels in chimeric mice and offsprings thereof.

For example, a target gene fragment wherein a Kozak sequence is located immediately upstream of the initiation codon can be inserted by homologous recombination into an exon containing the initiation codon of a myoglobin gene. In this case, the myoglobin gene of an allele having the target gene inserted therein is not expressed normally. However, since the myoglobin-gene-knock-out mouse is normal (Garry et al., Nature, 39: 5905-8, 1998), it is thought that non-expression of one allele of this gene does not have any effect on the phenotype of the chimeric mouse. Furthermore, in a manner similar to that in the above Example 1, IRES + Kozak sequence + target gene can be inserted within the 3' untranslated sequence located downstream of the termination codon of a myoglobin gene. In this case, a myoglobin gene of an allele having a target gene inserted therein can also be expressed.

A chimeric mouse can be produced by injecting ES cells altered as described above into an 8-cell-stage embryo obtained by the crossing of heterozygotes of a myogenin gene-disrupted mouse strain (Nabeshima *et al., supra*). There is a 25% probability of embryo being a homozygote (that is, deficient in the ability to form muscle tissue). Thus, in 25% of the chimeric mice that are born, muscle tissues are mainly derived from knock-in ES cells. Moreover, in the muscle cells of a mouse that have differentiated from knock-in ES cells, a foreign gene is expressed at high levels under the control of the regulatory sequence of a myoglobin gene.

### [Example 17] Construction of mouse FGF23 knock-out vector

### (1) Preparation of mouse FGF23 genomic region fragment

The following DNAs (primers) were synthesized from the nucleotide sequence encoding a mouse FGF23 (fibroblast growth factor 23) gene obtained from GenBank (NCBI, U.S.A.).
P51 : GACTCCTGGTGGGCGTGCTC (SEQ ID NO: 27)
P31: GGTGCCATCTACATGACCAT (SEQ ID NO: 28)

A reaction solution was prepared using Takara EX-Taq (TAKARA BIO INC., Japan) according to the instructions by the manufacturer. To 50 µL of the reaction solution, 10 pmol of each of the above primers and 25 ng of mouse TT2F cell-derived genomic DNA as a template were added. The solution was kept at 94°C for 2 minutes, and then subjected to 35 cycles of amplification, each cycle consisting of 94°C for 30 seconds, 60°C for 30 seconds, and 72°C for 20 seconds. PCR amplification fragments of 173 mer were collected from the obtained reaction solution by 2% agarose gel electrophoresis. Amplification fragments were collected from the excised gel using a QIAquick Gel Extraction Kit (QIAGEN, Germany) according to the instructions by the manufacturer. Thus, an FGF23P probe used for the selection of BAC clones containing the FGF23 genomic region was obtained.

### (2) Selection of BAC clones containing mouse FGF23 genomic region

BAC clones containing the FGF23 genomic region were screened by using a high density filter: BAC mouse C57/BL6 (KURABO, Japan) and the obtained FGF23P as a probe. As a result, 3 positive clones were obtained. The clone ID/address of these 3 types of clones were 17d5, 235I6, and 211K15. BAC clones were obtained based on the information on these positive clone numbers.

It was confirmed whether or not the obtained BAC clones containing the target FGF23 genomic region. To briefly explain this, a reaction solution was prepared using Takara EX Taq (TAKARA BIO INC., Japan) according to the instructions by the manufacturer. To 50 µL of the reaction solution, 10 pmol of each of P51 and P31 primers, and 100 ng of BAC clone DNA as a template were added. The solution was kept at 94°C for 2 minutes, and then subjected to 30 cycles of amplification, each cycle consisting of 94°C for 30 seconds, 60°C for 30 seconds, and 72°C for 20 seconds. The obtained reaction solution was analyzed by 2% agarose gel electrophoresis, a band of 173 mer was detected in case of clone numbers 235I6 and 211K15 had been used as templates. According to this result, it was confirmed that BAC clones of clone numbers 23516 and 211K15 contained the target FGF23 genomic region.

### (3) Subcloning of BAC clone to pBluescript II SK(-)

0.05 U of Sau3AI (Roche Diagnostics K. K., Japan) was added to 2.5 µg of DNA prepared from BAC clone of clone number 211K15, and the resultant was incubated at 37°C for 20 minutes. After separation on 0.8% gel, a band with a molecular weight of approximately 15 to 7 Kb was excised, and then DNA fragments were collected using a QIAquick Gel Extraction Kit (QIAGEN, Germany) according to the instructions by the manufacturer.

The above collected DNA fragments were inserted into pBluescript II SK(-) (TOYOBO, Japan) that had been digested with *Bam*H I, and then dephosphorylated with *Ercherichia coli* alkaline phosphatase. The resultant plasmid was then transfected into *Escherichia coli* MAX Efficiency STBL2 Competent Cells (Invitrogen). Clones wherein the DNA fragment had been inserted into the plasmid were selected using a plate supplemented with IPTG/X-gal, so that 100 white colonies were collected.

### (4) Selection of clones containing FGF23 genomic region

A reaction solution was prepared using Takara EX Taq (TAKARA BIO INC., Japan) according to the instructions. To 50 µL of the reaction solution, 10 pmol of each of P53 and P31 primers and 25 ng of plasmid DNA as a template were added. The solution was kept at 94°C for 2 minutes, and then subjected to 35 cycles of amplification, each cycle consisting of 94°C for 30 seconds, 60°C for 30 seconds, and 72°C for 20 seconds. Clones for which an amplification fragment of 173 mer was detected using 2% agarose gel electrophoresis were selected, thereby obtaining clone numbers 33 and 94.

### (5) Selection of clone containing a region of approximately 5 Kb located 5' upstream, and a region of approximately 2 Kb located 3' downstream, of FGF23 exon 1 region

KO53 and KO35 were synthesized from the nucleotide sequence of mouse FGF23 gene obtained from GenBank (NCBI, U.S.A.), and KO55 and KO33 were synthesized from the nucleotide sequence of pBluescript II SK (-).
KO55 : AATTAACCCTCACTAAAGGGAA (SEQ ID NO: 29)
KO53: CAAGCAATGGGGAAGTGTCTGG (SEQ ID NO: 30)
KO35: CGGCTACAGCCAGGACCAGCTA (SEQ ID NO: 31)
KO33: GTAATACGACTCACTATAGGGCCGA (SEQ ID NO: 32)

A reaction solution was prepared using KOD-Plus- (TOYOBO, Japan) according to the instructions by the manufacturer. To 50 µL of the reaction solution, in case of the target 5' region was selected, 10 pmol each of KO55 and KO53 primers, or in case of the target 3' region was selected, 10 pmol each of KO35 and KO33 primers, and 25 ng of plasmid DNA derived from clone number 33 or 94 as a template were added. The solution was kept at 94°C for 2 minutes, and then subjected to 30 cycles of amplification, each cycle consisting of 94°C for 15 seconds, 65°C for 20 seconds, and 68°C for 10 minutes. The size of the obtained amplification fragments were analyzed with 0.8% agarose gel. In case of the DNA prepared from clone number 33 was used as a template, approximately 5 Kb of a PCR amplification fragment was detected with a primer set for the selection of the 5' region, and approximately 2 Kb of a PCR amplification fragment was detected with the primer set for the selection of the 3' region. These results revealed that the target genomic region fragment had been subcloned into the clone of clone number 33.

### (6) Preparation of PCR amplification fragment 5' KO having restriction enzyme sites at both termini of 5' genomic homologous region

Using DNA derived from clone number 33, each nucleotide sequence of approximately 700 bp at both ends of the 5' upstream region of subcloned FGF23 exon 1 was determined. Based on the obtained information, approximately 5 Kb of 5' upstream region of FGF23 exon 1 was amplified, and a PCR primer set of NotI55 and FseI53, for the addition of the *Not* I site to the 5' side and the *Fse* I site to the 3' side of the amplification fragment, were designed.

NotI55: ATAAGAATGCGGCCGCTAAACTATAGCATCCACTGGGAATC
AACATCTGAGACATCCTA (SEQ ID NO: 33)

FseI53: CGGGCCGGCCCGCGGACTTTTAAAGGGTGGTGGTGTGAC
ATCAAGC (SEQ ID NO: 34)

A reaction solution was prepared using KOD-Plus- (TOYOBO, Japan) according to the instructions by the manufacturer. To 50 µL of the reaction solution, 10 pmol each of the above 2 primers and 25 ng of plasmid DNA of clone number 33 as a template were added. The solution was kept at 94°C for 2 minutes, and then subjected to 25 cycles of amplification, each cycle consisting of 94°C for 15 seconds and 68°C for 10 minutes. The thus obtained amplification fragments of approximately 5 Kb were separated and recovered using 0.8% agarose gel. Amplification fragments were collected from the collected gel using a QIAquick Gel Extraction Kit (QIAGEN, Germany) according to the instructions by the manufacturer. The collected PCR amplification fragments were digested with enzymes *Not* I and *Fse* I, and then separated and collected using 0.8% agarose gel. Enzyme-treated fragments were recovered from the collected gel using a QIAquick Gel Extraction Kit (QIAGEN, Germany) according to the instructions by the manufacturer.

### (7) Preparation of PCR amplification fragment 3'KO having restriction enzyme sites at both termini of the 3' genomic homologous region

Using DNA derived from clone number 33, each nucleotide sequence of approximately 700 bp at both ends of the 3' downstream region of subcloned FGF23 exon 1 was determined. Based on the obtained information, approximately 1.8 Kb of 3' downstream region of FGF23 exon 1 was amplified, and a PCR primer set of AscI55 and XhoI53, for the addition of the *Asc* I site to the 5' side and the *Xho* I site to the 3' side of the amplification fragment, were designed.

AscI55:    GGCGCGCCACTGCTAGAGCCTATCCAGACACTTCCCCA
TTGC (SEQ ID NO: 35)

XhoI53: CCGCTCGAGCGGTGTTCCAGACTGACCACCTTTCAACAA
AGAGATTC (SEQ ID NO: 36)

A reaction solution was prepared using KOD-Plus- (TOYOBO, Japan) according to the instructions by the manufacturer. To 50 µL of the reaction solution, 10 pmol each of the above 2 primers and 25 ng of plasmid DNA of clone number 33 as a template were added. The solution was kept at 94°C for 2 minutes, and then subjected to 25 cycles of amplification, each cycle consisting of 94°C for 15 seconds and 68°C for 10 minutes. The thus obtained amplification fragments with approximately 1.8 Kb were separated and collected using 0.8% agarose gel. Amplification fragments were recovered from the collected gel using a QIAquick Gel Extraction Kit (QIAGEN, Germany) according to the instructions by the manufacturer. The collected PCR amplification fragments were digested with restriction enzymes *Asc* I and *Xho* I, and then separated and collected using 0.8% agarose gel. Enzyme-treated fragments were recovered from the collected gel using QIAquick Gel Extraction Kit (QIAGEN, Germany) according to the instructions by the manufacturer.

### (8) Preparation of cassette vector pBlueLAB-LoxP-Neo-DT-A

The following DNAs were synthesized to add new restriction enzyme sites to a vector.
LinkA1:TCGAGTCGCGACACCGGCGGGCGCGCCC (SEQ ID NO: 37)
LinkA2:TCGAGGGCGCGCCCGCCGGTGTCGCGAC (SEQ ID NO: 38)
LinkB1:GGCCGCTTAATTAAGGCCGGCCGTCGACG (SEQ ID NO: 39)
LinkB2:AATTCGTCGACGGCCGGCCTTAATTAAGC (SEQ ID NO: 40)

A reaction solution wherein pBluescript II SK(-) (TOYOBO, Japan) had been treated with *Sal* I and *Xho* I restriction enzymes was subjected to phenol/chloroform extraction, and ethanol precipitation. To add the new restriction enzyme sites *Nru* I, *Sgr* A I and *Asc* I, to the plasmid, LinkA1 and LinkA2 were synthesized. A linker comprising the two oligo DNAs was inserted into the plasmid treated with restriction enzymes, and then the plasmid was transformed into *Escherichia coli* DH5α. DNA was prepared from the obtained transformant, so that a plasmid pBlueLA was obtained.

Subsequently, a reaction solution wherein PBlueLA had been treated with restriction enzymes *Not* I and *EcoR* I was subjected to phenol/chloroform extraction, and ethanol precipitation. To add new restriction enzyme sites *Pac* I, *Fse* I and *Sal* I to the plasmid, LinkB1 and LinkB2 were synthesized. A linker comprising the two oligo DNAs was inserted into the plasmid treated with restriction enzymes, and then the plasmid was transformed into *Escherichia coli* DH5α. DNA was prepared from the obtained transformant, so that a plasmid pBlueLAB was obtained.

The pLoxP-STneo plasmid described in the WO 00/10383 pamphlet (*supra*) was digested with *Xho* I, thereby obtaining a Neo-resistance gene (LoxP-Neo) having a LoxP sequence at both ends. Both ends of LoxP-Neo were blunt-ended using T4 DNA polymerase, so that LoxP-Neo-B was obtained.

After the above pBlueLAB was digested with *Eco*R V, the reaction solution was subjected to phenol/chloroform extraction and ethanol precipitation. After LoxP-Neo-B was inserted, the plasmid was transformed into *Escherichia coli* DH5α. DNA was prepared from the obtained transformant, so that a plasmid pBlueLAB-LoxP-Neo was obtained.

After pMC1DT-A (Invitrogen) was digested with *Xho* I and *Sal* I, the product was applied to 0.8% agarose gel. After a band of approximately 1 Kb was separated and collected, DT-A fragments were collected using a QIAquick Gel Extraction Kit (QIAGEN, Germany) according to the instructions by the manufacturer.

After pBlueLAB-LoxP-Neo was digested with *Xho* I, the reaction solution was subjected to phenol/chloroform extraction and ethanol precipitation. After DT-A fragment was inserted, the plasmid was transformed into *Eseherichica coli* DH5α. DNA was prepared from the obtained transformant, and a cassette vector pBlueLAB-LoxP-Neo-DT-A to be used for the preparation of a knock-out vector was obtained.

### (9) Construction of FGF 23 knock-out vector

After pBlueLAB-LoxP-Neo-DT-A was digested with *Asc* I and *Xho* I, the obtained DNA fragment of approximately 7.3 Kb was separated and purified using 0.8% agarose gel, and then dephosphorylated with *Escherichia coli* alkaline phosphatase. The genomic fragment 3' KO prepared in (7) was inserted into the fragment, and then the fragment was transformed into *Escherichia coli* MAX Efficiency STBL2 Competent Cells (Invitrogen). DNA was prepared from the obtained transformant. The nucleotide sequence of the inserted portion of the PCR amplification fragment was compared with the nucleotide sequence information obtained using clone number 33, thereby confirming that no amplification errors resulting from PCR amplification were contained, and confirming the nucleotide sequence of the ligation portion. Thus, a plasmid pBlueLAB-LoxP-Neo-DT-A-3'KO was obtained.

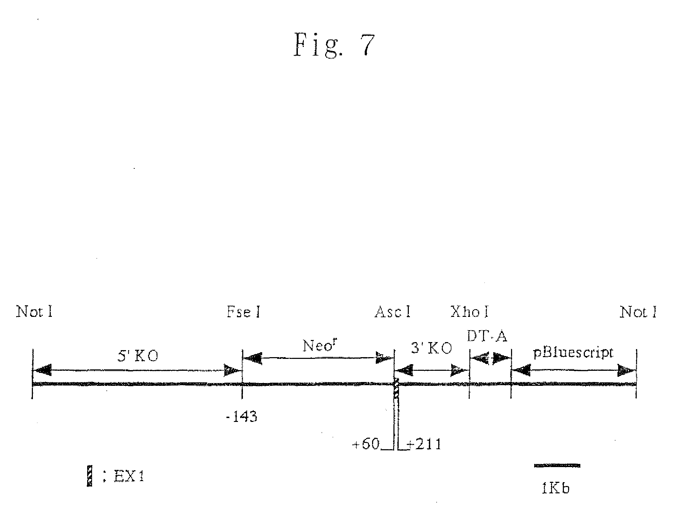

After pBlueLAB-LoxP-Neo-DT-A-3'KO was digested with *Not* I and *Fse* I, the obtained DNA fragment of approximately 9.1 Kb was separated and purified using 0.8% agarose gel, and then dephosphorylated with *Escherichia coli* alkaline phosphatase. The genomic fragment 5' KO prepared in (6) was inserted into the fragment, and then the product was transformed into *Escherichia coli* MAX Efficiency STBL4 Competent Cells (Invitrogen). DNA was prepared from the obtained transformant, and then the nucleotide sequence of the inserted portion of the PCR amplification fragment was compared with a region of approximately 3 Kb that had been obtained from the nucleotide sequence information obtained using clone number 33, thereby confirming that no amplification errors resulting from PCR amplification were contained within the range and confirming the nucleotide sequence of the ligation portion. Thus, a plasmid pBlueLAB-LoxP-Neo-DT-A-3'KO-5'KO was obtained. The structure of mouse FGF23 knock-out vector: pBlueLAB-LoxP-Neo-DT-A-3'KO-5'KO is shown in Fig. 7.

### (10) Preparation of FGF23 knock-out vector for electroporation

Using a buffer (Roche Diagnostics, Japan, H buffer for restriction enzyme) supplemented with spermidine (1 mM, pH 7.0, SIGMA U.S.A.), 150 µg of pBlueLAB-LoxP-Neo-DT-A-3'KO-5'KO was digested with *Not* I or *Xho* I at 37°C for 5 hours. After phenol/chloroform extraction, 2.5 volumes of 100% ethanol and 0.1 volume of 3M sodium acetate were added, and then the solution was kept at -20°C for 16 hours. The linearized vector digested with *Not* I or *Xho* I was collected by centrifugation, and then sterilized by the addition of 70% ethanol. 70% ethanol was removed in a clean bench and then air-dried for 1 hour. An HBS solution was added to result in a 0.5 µg/µL DNA solution and the resultant was stored at room temperature for 1 hour, so that FGF23 knock-out vectors for electroporation, FGF-KO-NotI and FGF-KO-XhoI were prepared. FGF-KO-NotI and FGF-KO-XhoI have termini shown with *Not* I and *Xho* I, respectively, in Fig. 7.

### (11) Preparation of probe for genomic Southern analysis

The following DNAs for obtaining oligo DNA containing a region of 525 mer immediately downstream of the 3' KO were synthesized based on the nucleotide sequence information using DNA derived from clone number 33.
OST3S5 : TCAGTCTI1AATGGCAGGCTTACAGACATCC (SEQ ID NO: 41)
OST3S3: TGAGGCAGATCATTCCATCTTGTCAAGACC (SEQ ID NO: 42)

A reaction solution was prepared using Takara EX Taq (TAKARA BIO INC., Japan) according to the instructions by the manufacturer. To 50 µL of the reaction solution, 10 pmol of each of the above 2 primers and 25 ng of DNA derived from BAC of clone number 211K15 as a template were added. The solution was kept at 94°C for 2 minutes, and then subjected to 33 cycles of amplification, each cycle consisting of 94°C for 30 seconds, 60°C for 30 seconds, and 72°C for 1 minute. The obtained 544 mer of amplification fragments were separated and collected using 0.8% agarose gel. From the collected gel, a 3' genomic probe for the Southern, 3' KO-prob, was collected using a QIAquick Gel Extraction Kit (QIAGEN, Germany) according to the instructions by the manufacturer.

### [Example 18] Preparation of FGF23 knock-out ES cell clone

To obtain a mouse FGF23 knock-out ES cell line by homologous recombination, the pBlueLAB-LoxP-Neo-DT-A-3'KO-5'KO prepared in Example 17 was linearized with a restriction enzyme *Not* I or *Xho* I (TAKARA BIO INC., Japan), and then transfected into a mouse ES cell TT2F (Yagi et al., Analytical Biochem., 214: 70, 1993) according to the established method (Shinichi Aizawa. Bio Manual Series 8, Gene Targeting, YODOSHA, 1995).

The method for culturing TT2F was performed according to the above-described method (Shinichi Aizawa, *supra*). Feeder cells used herein were G418-resistant primary cultured cells (purchased from Invitrogen, Japan) treated with mitomycin C (SIGMA, U.S.A.). First, the propagated TT2F cells were trypsinized, and then suspended in HBS to result in a 3×10⁷ cells/ml suspension. 0.5 ml of the cell suspension was mixed with 10 µg of the vector DNA, and the resultant was subjected to electroporation using a Gene Pulser Cuvette (electrode distance: 0.4 cm, Bio-Rad, U.S.A.) (capacity: 960 µF; voltage: 240 V; room temperature). The cells that had been subjected to electroporation were suspended in 10 ml of an ES medium, and then plated onto a single 100 mm plastic dish for tissue culture (Falcon, Becton Dickinson, U.S.A.) onto which feeder cells had been previously inoculated. 36 hours later, the medium was changed with an ES medium containing 0.8 µg/ml puromycin (SIGMA, U.S.A.). Colonies that had appeared 7 days later were picked up, and each colony was allowed to grow to reach confluence in a 24-well plate. Two-thirds of the colonies were suspended in 0.2 ml of a medium for storage (ES medium +10% DMSO, SIGMA, U.S.A.), and then stored at -80°C. The remaining one-third of the colonies were inoculated onto a 12-well gelatine- coated plate, and then cultured for 2 days. Then, genomic DNA was prepared from 10⁶-10⁷ cells using Puregene DNA Isolation Kits (Gentra Systems, U.S.A.). These G puromycin-resistant TT2F cell genomic DNAs were digested with a restriction enzyme *Hind* III (TAKARA BIO INC., Japan), and then separated on 0.8% agarose gel electrophoresis. Subsequently, Southern blot was performed, and then homologous recombinants were detected using as a probe a DNA fragment (3' KO-prob; see Example 17 (1)) located in a downstream region immediately following the 3' homologous region of the knock-out vector. In wild-type TT2F cells, 2 bands (approximately 6 Kb and approximately 4.5 Kb) were detected by digestion with *Hin*d III. It was predicted that in the homologous recombinants, the band of approximately 6 Kb would disappear and instead, a new band of approximately 2.5 Kb would appear. In the neomycin-resistant clone, a new band of approximately 2.5 Kb was confirmed. Hence, in these clones, a neomycin-resistance gene (including the restriction enzyme sites derived from the knock-out vector at both of its ends) had been inserted into a region of the initiation codon of the FGF23 gene (a range between -142 and +59, when the position of A of ATC corresponding to the initiation methionine is supposed to be +1, and a position located 1 nucleotide upstream from this A is supposed to be -1) of one of the alleles. As a result of Southern analysis, when pBlueLAB-LoxP-Neo-DT-A-3'KO-5'KO was linearized with a restriction enzyme *Not* I, 16 out of 90 clones (18%) were homologous recombinants, and when pBlueLAB-LoxP-Neo-DT-A-3'KO-5'KO was linearized with a restriction enzyme *Xho* I, 1 out of 28 clones (3.6%) were homologous recombinants. These results showed that, when a targeting vector is linearized, a case where negative selection marker gene DT-A is not located at the terminus of the vector structure is more advantageous in homologous recombination compared with a case where the same is located at the terminus (see also Fig. 7). In cases where 6 different types of knock-out vectors having structures similar to the above cases were used for each different gene, an average homologous recombination efficiency of 22.3%, which was higher than generally reported homologous recombination efficiencies (approximately several percent), could be obtained.

All publications, patents, and patent applications cited herein are incorporated herein by reference in their entirety.

### INDUSTRIAL APPLICABILITY

According to the present invention, chimeric non-human animals (e.g., chimeric mice) can be obtained expressing at high levels a desired protein efficiently and reliably compared with conventional methods. Since an embryo used as a host embryo in the present invention is deficient in a cell and/or tissue wherein an introduced gene encoding a desired protein is expressed, all the cells and/or tissues in the thus produced chimeric non-human animal are all derived from pluripotent cells containing the introduced nucleic acid sequence (structural gene), so that the desired protein can be expressed at a high efficiency. In the present invention, preferably the expression system of an immunoglobulin light chain, and particularly preferably the expression system of a κ chain, is utilized. The homologous recombination efficiency in this Igκ gene locus is 5% or more, which is higher than those of conventional methods. Therefore, the present invention can be used for the production of a desired protein by the high-level expression of a gene encoding the desired protein, or for the analysis of *in vivo* functions of a gene or a protein with unknown functions.

### SEQUENCE FREE TEXT

SEQ ID NOS: 1 to 6: synthetic oligonucleotide
SEQ ID NOS: 7 and 8: *Sal* I recognition sequence
SEQ ID NOS: 9 to 20: synthetic oligonucleotide
SEQ ID NOS: 21 and 22: multi-cloning site
SEQ ID NOS: 23 to 26: synthetic oligonucleotide
SEQ ID NO: 29: synthetic oligonucleotide
SEQ ID NOS: 32 to 40: synthetic oligonucleotide

## Claims

1. A method for analyzing the in vivo functions of a desired protein or a gene encoding the desired protein, which comprises
preparing a chimeric non-human animal, which is derived from a pluripotent cell derived from a non-human animal containing a genome wherein a nucleic acid sequence encoding a desired protein is located so that the expression of the desired protein is regulated by the regulatory region of a gene that is expressed in a specific cell and/or tissue and a host embryo of a non-human animal strain deficient in the specific cell and/or tissue, and is capable of expressing the desired protein in at least the specific cells and/or tissues; and
comparing the phenotype of the chimeric non-human animal or an offspring thereof with that of a corresponding wild-type non-human animal containing no nucleic acid sequence encoding the desired protein, so as to determine differences in these phenotypes.

2. The method of claim 1, wherein the nucleic acid sequence encoding a desired protein is located downstream of the regulatory region of a gene that is expressed in a specific cell and/or tissue.

3. The method of claim 1, wherein the nucleic acid sequence encoding a desired protein is located downstream of an internal ribosomal entry site located downstream of a termination codon of a gene that is expressed in a specific cell and/or tissue.

4. The method of claim 1, wherein a sequence containing the nucleic sequence encoding a desired protein is located between the termination codon of a gene that is expressed in a specific cell and/or tissue and a polyA signal region.

5. The method of claim 1, wherein a sequence containing a second polyA signal region, a promoter sequence, and the nucleic acid sequence encoding a desired protein is located between the termination codon of a gene that is expressed in a specific cell and/or tissue and a polyA signal region.

6. The method of claim 5, wherein the promoter sequence is derived from a gene that is expressed in a specific cell and/or tissue.

7. The method of claim 1, wherein a sequence containing a promoter sequence, the nucleic acid sequence encoding a desired protein, and a second polyA signal region is located downstream of a polyA signal region.

8. The method of claim 7, wherein the promoter sequence is derived from a gene that is expressed in a specific cell and/or tissue.

9. The method of claim 7, wherein the distance between the polyA signal region and the promoter sequence is less than 1 Kb.

10. The method of any one of claims 1 to 9, wherein the pluripotent cell contains both a genome wherein the nucleic acid sequence encoding a desired protein is located so that the expression of the desired protein is regulated by the regulatory region of a gene that is expressed in at least a specific cell and/or tissue, and a genome wherein the allele of the gene that is expressed in the specific cell and/or tissue is inactivated.

11. The method of any one of claims 1 to 10, wherein the pluripotent cell is an embryonic stem cell.

12. The method of any one of claims 1 to 11, wherein the chimeric non-human animal is selected from the group consisting of mice, cattle, pigs, monkeys, rats, sheep, goats, rabbits, and hamsters.

13. The method of claim 12, wherein the chimeric non-human animal is a mouse.

14. The method of any one of claims 1 to 13, wherein a combination of a gene that is expressed in a specific cell and/or tissue and the cell and/or tissue deficient in a non-human animal strain is selected from the group consisting of the following (1) to (7):
(1) an immunoglobulin light chain or heavy chain gene and a B-lymphocyte
(2) a T-cell receptor gene and a T-lymphocyte;
(3) a myoglobin gene and a muscle cell;
(4) a crystallin gene and a crystalline lens of an eyeball;
(5) a renin gene and a kidney tissue;
(6) an albumin gene and a liver tissue; and
(7) a lipase gene and a pancreas tissue.

15. The method of claim 14, wherein the combination of a gene that is expressed in a specific cell and/or tissue and the specific cell and/or tissue deficient in a non-human animal strain is that of an immunoglobulin light chain κ gene and a B-lymphocyte.

16. A method for producing a desired protein, comprising preparing a chimeric non-human animal, which is derived from a pluripotent cell derived from a non-human animal containing a genome wherein a nucleic acid sequence encoding a desired protein is located so that the expression of the desired protein is regulated by the regulatory region of a gene that is expressed in a specific cell and/or tissue and a host embryo of a non-human animal strain deficient in the specific cell and/or tissue, and is capable of expressing the desired protein in at least the specific cells and/or tissues;
causing the production of the desired protein using the chimeric non-human animals, a tissue or cell of the chimeric non-human animal, or a hybridoma that is produced by the fusion of a cell of the chimeric non-human animal and a proliferable tumor cell; and
collecting the proteins.

17. The method of claim 16, wherein the chimeric non-human animal is selected from the group consisting of mice, cattle, pigs, monkeys, rats, sheep, goats, rabbits, and hamsters.

18. The method of claim 17, wherein the chimeric non-human animal is a mouse.

19. The method of any one of claims 16 to 18, wherein a combination of a gene that is expressed in a specific cell and/or tissue and the specific cell and/or tissue deficient in a non-human animal strain is selected from the group consisting of the following (1) to (7):
(1) an immunoglobulin light chain or heavy chain gene and a B-lymphocyte
(2) a T-cell receptor gene and a T-lymphocyte;
(3) a myoglobin gene and a muscle cell;
(4) a crystallin gene and a crystalline lens of an eyeball;
(5) a renin gene and a kidney tissue;
(6) an albumin gene and a liver tissue; and
(7) a lipase gene and a pancreas tissue.

20. The method of claim 19, wherein the combination of a gene that is expressed in a specific cell and/or tissue and the specific cell and/or tissue deficient in a non-human animal strain is that of an immunoglobulin light chain κ gene and a B-lymphocyte.
